# EUROPEAN PATENT APPLICATION

(11) **EP 1 803 758 A1**
(43) Date of publication of application: **04.07.2007**
(21) Application number: 05795841.5
(22) Date of filing: 19.10.2005
(51) Int. Cl.: C08G 59/02, C08G 59/20, C07D 303/30

(54) **POLYEPOXY COMPOUND, METHOD FOR PRODUCING SAME, THERMOSETTING RESIN COMPOSITION CONTAINING SAME, CURED PRODUCT OF SUCH COMPOSITION, AND METHOD FOR REMOVING SUCH CURED PRODUCT**

(30) Priority: 20.10.2004 JP 2004305017; 20.10.2004 JP 2004305018; 20.10.2004 JP 2004305019
(71) Applicant: KANSAI PAINT CO., LTD., Amagasaki-shi, Hyogo-ken 661-8555 (JP)
(72) Inventor: IWASHIMA, Chiaki, c/o KANSAI PAINT CO., LTD.,, Hiratsuka-shi, Kanagawa 2548562 (JP); KOBATA, Masami, c/o KANSAI PAINT CO., LTD.,, Hiratsuka-shi, Kanagawa 2548562 (JP); IMAI, Genji, c/o KANSAI PAINT CO., LTD., 17-1,, Hiratsuka-shi, Kanagawa 2548562 (JP)
(74) Representative: Kuhnen & Wacker
(86) International application number: PCT/JP2005/019240
(87) International publication number: WO 2006/043608

(57) **Abstract**

The present invention provides a specific polyepoxy compound having at least two olefin oxide groups and at least one tertiary ester group per molecule, a method for producing the compound, a thermosetting resin composition containing the compound, a cured product of the composition, and a method for removing the cured product.

## Description

### TECHNICAL FIELD

The present invention relates to a polyepoxy compound, a method for producing the polyepoxy compound, a thermosetting resin composition comprising the polyepoxy compound, and a cured product of the composition, and a method for removing the cured product.

### BACKGROUND ART

It has been desired from the viewpoint of resource recycling that after scrapping automobiles, resin materials used for the automobile exterior parts (e.g., polypropylene resins used for bumpers), etc. should be reverted to raw materials and reused. It has also been desired that after scrapping, metal materials of, for example, automobile exterior parts, personal computers and like electronic equipment, canned products, automobile bodies, etc. should be reverted to raw materials and reused.

When resin scraps, metal scraps, etc. are reused, it is necessary to remove coating film, etc. formed thereon to prevent deterioration in the quality of recycled products.

Conventional methods for removing such a coating film from coated resin scraps include, for example, melting separation methods, comprising melting pulverized scraps by heating, followed by filtration to separate a liquid-state substrate thermoplastic resin from a solid-state thermosetting coating film; and blasting methods, comprising ejecting a powder such as sands or a liquid such as water onto the coated scrap surface at a high velocity and high pressure to give impact thereto and thereby remove the coating film.

Japanese Unexamined Patent Publication No. 1997-11237 discloses a recycling method comprising subjecting coated resin scraps to 1,000 °C or higher frame treatment to carbonize only the coating film into carbon black to render the scraps harmless, then pulverizing, re-melting and reverting the scraps to a raw material, and forming the raw material into a product.

Japanese Unexamined Patent Publication No. 2003-200349 discloses a blasting method for removing a coating film from coated nonferrous metal scraps that comprises making cuts on the coated film of the scraps and ejecting an ejection material such as plastic grains, vegetable grains, etc. at a high velocity onto the coated surface to remove the coating film.

However, these conventional methods for removing a coating film from resin scraps, nonferrous metal scraps, etc. have problems in that the methods comprise complicated steps and require special equipment.

WO00/56799 discloses a recyclable thermosetting resin composition comprising an alicyclic epoxy compound containing a thermally cleavable bond, and a curing agent such as an anhydride compound or an amine compound. The cured product of the composition can be decomposed and removed by heating the cured product to a temperature higher than the curing temperature of the composition. Representative examples of the epoxy compound used in the composition include epoxy compounds represented by the following formula:

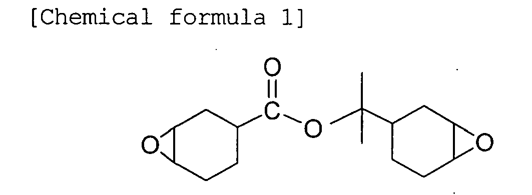

However, the alicyclic epoxy group of the above epoxy compound has a problem in that it is difficult to cleave the ring and has low reactivity with commonly used curing agents for epoxy resins. Therefore, the above disclosed thermosetting resin composition has insufficient curability.

### DISCLOSURE OF THE INVENTION

### PROBLEM TO BE SOLVED BY THE INVENTION

An object of the present invention is to provide a polyepoxy compound that can be decomposed and removed by post-heating a cured product thereof.

Another object of the invention is to provide a method for producing the polyepoxy compound.

A further object of the invention is to provide a thermosetting resin composition containing the polyepoxy compound.

Still another object of the invention is to provide a cured product of the composition and a method for removing the cured product.

Other objects and features of the invention will become apparent from the following detailed description.

### MEANS FOR SOLVING THE PROBLEMS

The present inventors carried out extensive research and found that the above objects can be achieved by a specific polyepoxy compound containing at least two olefin oxide groups and at least one tertiary ester group per molecule. The inventors carried out further research based on this finding and have accomplished the present invention.

The present invention provides the following polyepoxy compounds, methods of producing such a polyepoxy compound, thermosetting resin compositions comprising such a polyepoxy compound, cured products of such compositions, and methods of removing the cured products.
1. A polyepoxy compound containing at least two olefin oxide groups and at least one tertiary ester group per molecule and having a weight average molecular weight of 100 to 50,000, the tertiary ester group being represented by general formula (I). 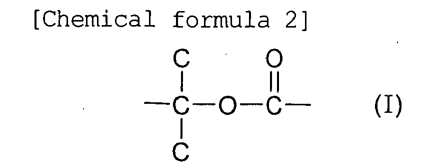
2. A polyepoxy compound according to item 1 which is represented by general formula (II) 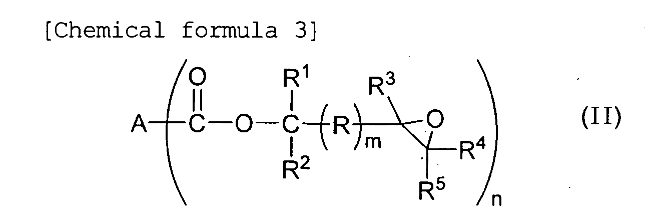 wherein n is an integer from 2 to 8, m is an integer of 0 or 1, R¹ and R² each independently represent a C₁₋₂₄ monovalent organic group, R³, R and R each independently represent a hydrogen atom, a C₁₋₆ lower alkyl group, a C₁₋₄ lower alkoxy group, a halogen atom, a cyano group, or a nitro group, R is a C₁₋₂₄ divalent organic group, and A is a C₁₋₂₄ divalent to octavalent organic group.
3. A polyepoxy compound according to item 2 wherein R is an organic group containing ether bond(s).
4. A polyepoxy compound according to item 1 which is at least one compound selected from the group consisting of polyepoxy compounds of the following formulas 1 to 5: 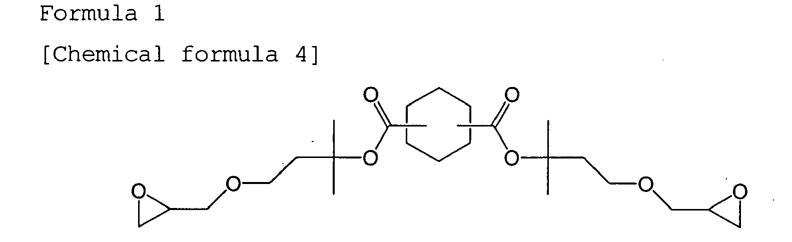 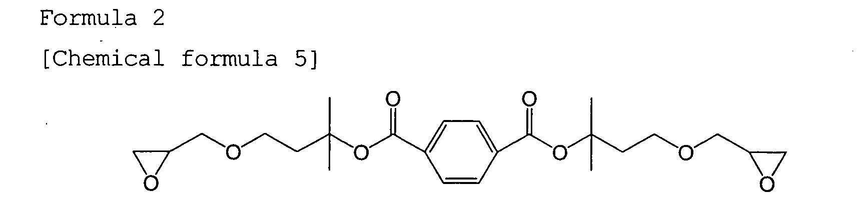 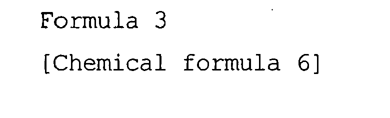 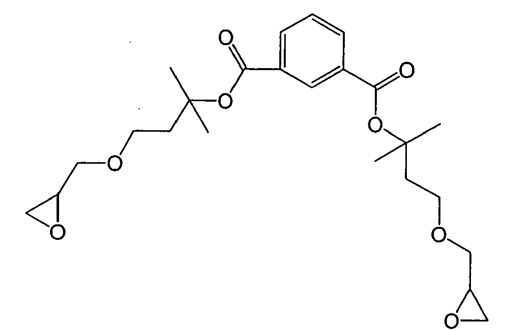 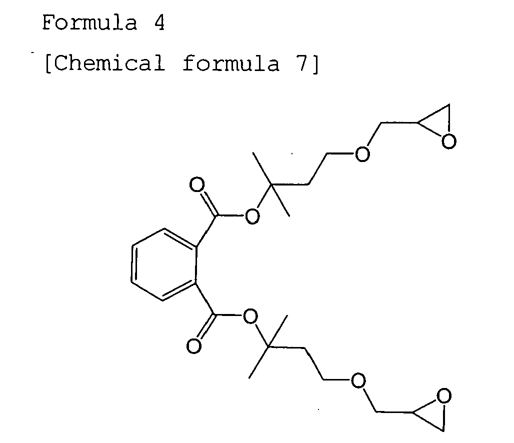 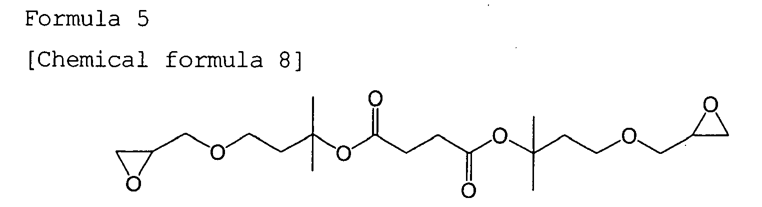
5. A method for producing a polyepoxy compound, the method comprising esterifying at least one compound selected from the group consisting of:
   (a) compounds containing per molecule at least two haloformyl groups represented by general formula (III) 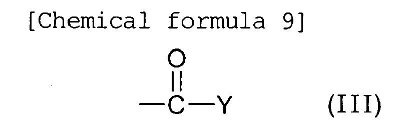 wherein Y is a halogen atom; (b) polycarboxylic acids; and (c) acid anhydrides; with a tertiary alcohol containing an olefin bond to produce an intermediate containing olefin bonds and tertiary ester bonds,
      then oxidizing the olefin bonds of the intermediate to produce a compound containing olefin oxide groups and tertiary ester groups.
6. A method according to item 5 wherein the tertiary alcohol containing an olefin bond is a compound represented by general formula (IV) 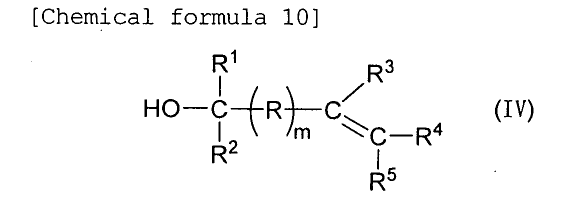 wherein n is an integer from 2 to 8, m is an integer of 0 or 1, R¹ and R² each independently represent a C₁₋₂₄ monovalent organic group, R³, R⁴ and R⁵ each independently represent a hydrogen atom, a C₁₋₆ lower alkyl group, a C₁₋₄ lower alkoxy group, a halogen atom, a cyano group, or a nitro group, and R is a C₁₋₂₄ divalent organic group.
7. A method according to item 6 wherein the tertiary alcohol containing an olefin bond is an allyl ether compound represented by general formula (V) 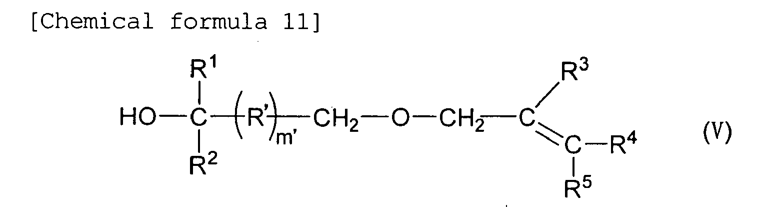 wherein m' is an integer of 0 or 1, R' is a C₁₋₂₂ divalent organic group, R¹, R², R³, R⁴ and R⁵ are as defined above.
8. A thermosetting resin composition comprising (A) a carboxyl- and/or hydroxyphenyl-containing resin, and (B) the polyepoxy compound of item 1.
9. The thermosetting resin composition according to item 8 wherein the resin (A) is at least one resin selected from the group consisting of:
   carboxyl-containing resin (a) having a weight average molecular weight of 500 to 100,000, a glass transition point of 0 to 200°C, and an acid value of 5 to 700 mgKOH/g;
   hydroxyphenyl-containing resin (b) having a weight average molecular weight of 500 to 100,000, a glass transition point of 0 to 200°C, and a hydroxyl value of 5 to 600 mgKOH/g; and
   carboxyl- and hydroxyphenyl-containing resin (c) having a weight average molecular weight of 500 to 100,000, a glass transition point of 0 to 200°C, an acid value of 5 to 700 mgKOH/g, and a hydroxyl value of 5 to 600 mgKOH/g.
10. The thermosetting resin composition according to item 8 which comprises 1 to 3,000 parts by weight of the compound (B) per 100 parts by weight of the resin (A).
11. A cured product obtained by heating and curing the thermosetting resin composition of item 8.
12. A method for removing a cured product, the method comprising heating the whole or part of the cured product of item 11 at a temperature higher than the heat-curing temperature to thermally cleave a tertiary ester bond derived from the polyepoxy compound (B), and removing the heated portion of the cured product by using or not using a treatment agent.
13. The method for removing the cured product of item 12 wherein the treatment agent is an organic solvent.
14. The method for removing the cured product of item 12 wherein the cured product is a cured coating film or cured adhesive film formed on scrap.
15. A thermosetting resin composition comprising (B) the polyepoxy compound of item 1, and (C) a curing agent.
16. The thermosetting resin composition according to item 15 wherein the curing agent (C) is at least one compound selected from the group consisting of acid anhydrides, polycarboxylic acids, polyphenolic compounds, nitrogen-containing compounds, cationic polymerization catalysts, and sulfur-containing compounds.
17. The thermosetting resin composition according to item 16 wherein the acid anhydride is at least one compound selected from the group consisting of hexahydrophthalic anhydride, methylhexahydrophthalic anhydride, methylnadic anhydride, and maleic anhydride.
18. The thermosetting resin composition according to item 16 wherein the polycarboxylic acid is at least one compound selected from the group consisting of phthalic acid, isophthalic acid, terephthalic acid, and 4,4'-biphenyldicarboxylic acid.
19. The thermosetting resin composition according to item 16 wherein the polyphenolic compound is at least one compound selected from the group consisting of resorcinol and bisphenols.
20. The thermosetting resin composition according to item 16 wherein the nitrogen-containing compound is at least one compound selected from the group consisting of amine compounds, amide compounds, and imidazole compounds.
21. The thermosetting resin composition according to item 16 wherein the cationic polymerization catalyst is at least one compound selected from the group consisting of Lewis acid salts of aromatic diazonium, Lewis acid salts of aromatic sulfonium, Lewis acid salts of aromatic iodonium, and Lewis acid salts of metallocene compounds.
22. The thermosetting resin composition according to item 15 comprising 0.1 to 500 parts by weight of curing agent (C) per 100 parts by weight of polyepoxy compound (B).
23. The cured product obtained by heating and curing the thermosetting resin composition of item 15.
24. A method for removing a cured product, the method comprising heating the whole or part of the cured product of item 23 at a temperature higher than the heat-curing temperature to thermally cleave a tertiary ester bond derived from the polyepoxy compound (B), and removing the heated portion of the cured product by using or not using a treatment agent.
25. The method according to item 24 wherein the treatment agent is an organic solvent.
26. The method according to item 24 wherein the cured product is a cured coating film or cured adhesive film formed on scrap.

### Polyepoxy compound

The polyepoxy compound of the invention contains at least two olefin oxide groups and at least one tertiary ester group represented by general formula (I) 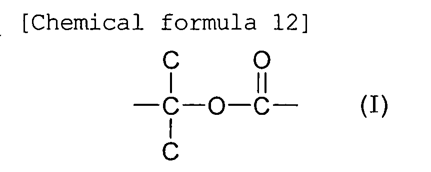 per molecule, and has a weight average molecular weight of about 100 to about 50,000. The polyepoxy compound preferably contains 2 to 8 olefin oxide groups and 2 to 8 tertiary ester groups and a weight average molecular weight of about 200 to about 45,000.

When the polyepoxy compound does not contain at least 2 olefin oxide groups, a cured product of a composition containing such a polyepoxy compound tends to have reduced strength. When the polyepoxy compound does not contain at least one tertiary ester group, the cured product of a composition containing such a polyepoxy compound tends to exhibit low degradability by post-heating, so the cured product after post-heating tends to have low solubility in treatment agents such as organic solvents, thus being difficult to remove. When the polyepoxy compound has a weight average molecular weight of less than 100, the polyepoxy compound has a high volatility, so that the polyepoxy compound evaporates upon heating and results in reduced concentration of the polyepoxy compound in the composition, thus being difficult to cure by heating. When the polyepoxy compound has a weight average molecular weight of more than 50,000, the cured product after post-heating tends to have low solubility in treatment agents such as organic solvents, thus being difficult to remove.

In the present invention, the term "olefin oxide group" refers to a functional group obtained by oxidizing an olefinic double bond to form an epoxy group.

The polyepoxy compound of the invention is preferably a compound represented by the following general formula (II) 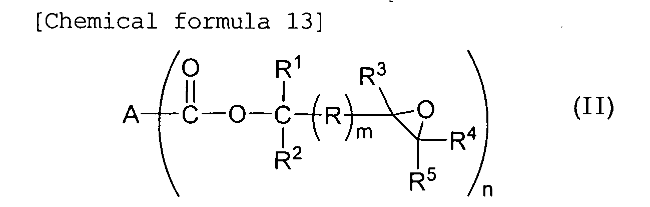 wherein n is an integer from 2 to 8, m is an integer of 0 or 1, R¹ and R² each independently represent a C₁₋₂₄ monovalent organic group, R³, R⁴ and R⁵ each independently represent a hydrogen atom, a C₁₋₆ lower alkyl group, a C₁₋₄ lower alkoxy group, a halogen atom, a cyano group, or a nitro group, R is a C₁₋₂₄ divalent organic group, and A is a C₁₋₂₄ divalent to octavalent organic group.

A compound of general formula (II) wherein R is an organic group containing ether bond(s) is particularly preferable as the polyepoxy compound of the invention in view of the excellent reactivity of the olefin oxide groups. Methylglycidyl and glycidyl groups are preferable as olefin oxide groups in view of the excellent reactivity of the olefin oxide groups.

In general formula (II), the organic groups represented by R¹, R² and R may contain atoms other than carbon such as heteroatoms, and R¹ and R² should be each independently bonded via a carbon atom to the carbon atom of -O-C to form a tertiary ester group.

In general formula (II), C₁₋₂₄, i.e., the number of carbon atoms of a monovalent or divalent organic group represented by R¹, R² and R refers to the total number of carbon atoms contained in the organic group. For example, when the organic group comprises an aliphatic hydrocarbon group and an aromatic hydrocarbon group bonded together, the number of carbon atoms refers to the total number of carbon atoms in the aliphatic hydrocarbon group and aromatic hydrocarbon group (including carbon atoms of substituents thereof). When heteroatoms, etc. are present in the carbon chain, the number of carbon atoms refers to the total number of carbon atoms including the number of carbon atoms in the carbon chain containing the heteroatom(s) and the number of carbon atoms that form heterocyclic ring(s) with the heteroatom(s).

In general formula (II), examples of C₁₋₂₄ monovalent organic groups represented by R¹ and R² include aliphatic hydrocarbon groups, aromatic hydrocarbon groups, alicyclic hydrocarbon groups, and combinations thereof.

Examples of aliphatic hydrocarbon groups include C₁₋₆ lower alkyl groups, such as methyl, ethyl, n-propyl, isopropyl, n-butyl, isobutyl, t-butyl, s-butyl, n-pentyl, isopentyl, t-pentyl, neopentyl, n-hexyl, isohexyl, etc.; and alkyl groups containing 7 or more carbon atoms, such as octyl, nonyl, decyl, dodecyl, tetradecyl, etc. Such groups may be linear or branched.

Such alkyl groups may be halogenated alkyl groups obtained by substitution with halogens. Examples of halogenated alkyl groups include fluoropropyl, chloropropyl, fluorobutyl, trifluoropropyl, etc.

Examples of halogen atoms usable in the invention include fluorine, chlorine, bromine, and iodine.

Aromatic hydrocarbon groups may be monocyclic or polycyclic, and aryl can be stated as an example. Examples of monocyclic aromatic hydrocarbon groups include phenyl, toluyl, xylyl, etc. Examples of polycyclic aromatic hydrocarbon groups include naphthyl, etc. Aromatic hydrocarbon groups may be those in which some or all of the hydrogen atoms bonded to the aromatic ring may be substituted with C₁₋₆ lower alkyl groups, halogen atoms, etc. Particularly preferable are phenyl, phenyl substituted with methyl, etc.

Usable alicyclic hydrocarbon groups generally have 3 to 7 carbon atoms. Examples thereof include cyclopropyl, cyclobutyl, cyclopentyl, cyclohexyl, cyclohexenyl, cycloheptyl, etc., and cyclohexyl is particularly preferable.

In general formula (II), examples of heteroatom-containing C₁₋₂₄ monovalent organic groups represented by R¹ and R² include aliphatic hydrocarbon groups having heteroatom(s) bonded to end or middle carbon(s) of the chain. Examples of such heteroatom-containing organic groups include organic groups having bonded thereto a compound such as furan, thiophene, pyrrol, pyridine, cyclic ether, lactone, cyclic imine, lactam, etc.

According to the invention, compounds of general formula (II) wherein R¹ and R² designate methyl groups are particularly preferable in view of the excellent thermal cleavability of the tertiary ester bonds.

In general formula (II), examples of C₁₋₆ lower alkyl groups represented by R³, R⁴ and R⁵ include the C₁₋₆ lower alkyl groups mentioned above.

In general formula (II), examples of C₁₋₄ lower alkoxy groups represented by R³, R⁴ and R⁵ include methoxy, ethoxy, n-propoxy, isopropoxy, n-butoxy, isobutoxy, t-butoxy, s-butoxy, etc. The groups represented by R³, R⁴ and R⁵ are preferably hydrogen or methyl, and more preferably hydrogen, because such a polyepoxy compound of general formula (II) has excellent reactivity.

In general formula (II), examples of C₁₋₂₄ divalent organic groups represented by R include aliphatic hydrocarbons, aromatic hydrocarbons, alicyclic hydrocarbons, and combinations thereof. Some or all of the hydrogen atoms bonded to the aromatic ring may be substituted with C₁₋₆ lower alkyl groups and halogen atoms. Some or all of the hydrogen atoms of the aliphatic hydrocarbon may be substituted with halogen atoms.

Specific examples of groups represented by R include the following.

Examples of divalent aromatic hydrocarbons include the following: 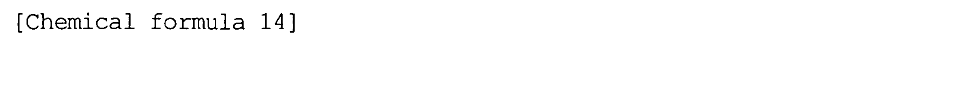 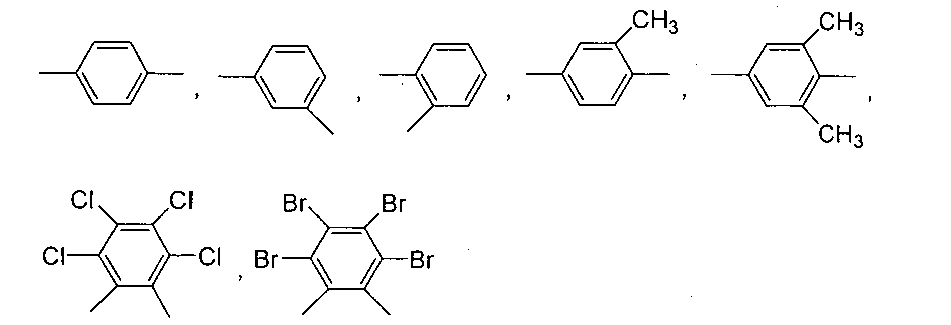 , etc.

Examples of divalent aliphatic hydrocarbons include -CH₂-, -C₂H₄-, -C₃H₆-, -C₄H₈-, -C₅H₁₀-, -C₆H₁₂-, -C₁₀H₂₀-, -C₁₂H₂₄-, -C₁₆H₃₂-, etc.

Examples of divalent alicyclic hydrocarbons include the following: 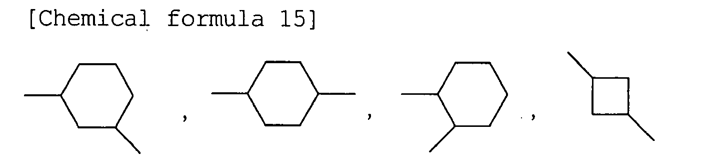 , etc.

The organic group represented by R may be a combination of aliphatic hydrocarbon and aromatic hydrocarbon or alicyclic hydrocarbon. Examples thereof include the following: 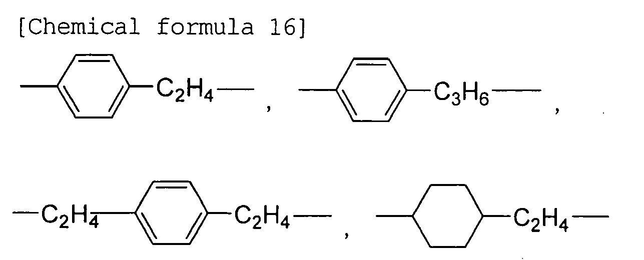 , etc.

In general formula (II), examples of heteroatoms that can be contained in C₁₋₂₄ divalent organic groups represented by R include atoms other than carbon, such as oxygen, sulfur, nitrogen, etc. Such a heteroatom can be contained in the C₁₋₂₄ divalent organic group as a single atom, or a group formed with other atom(s), for example, a linear group such as an ether, ester, thioether, amide, etc., a heterocycylic ring formed with carbon atom(s), etc. Such a heterocyclic ring is contained in the organic group as the residue of a heterocyclic ring introduced in the form of a compound such as furan, thiophene, pyrrol, pyridine, cyclic ether, lactone, cyclic imine, lactam, etc.

Examples of heteroatom-containing divalent organic groups represented by R include aliphatic hydrocarbons, aromatic hydrocarbons, and alicyclic hydrocarbons, each having a heteroatom bonded to an end thereof; aliphatic hydrocarbons bonded to each other via a heteroatom, an aliphatic hydrocarbon and an aromatic or alicyclic hydrocarbon bonded to each other via a heteroatom, or an aromatic hydrocarbon and an aliphatic hydrocarbon bonded to each other via a heteroatom, etc.

Examples of C₁₋₂₀ linear or branched divalent organic groups which may contain an ether, ester, thioether or amide group include the following: 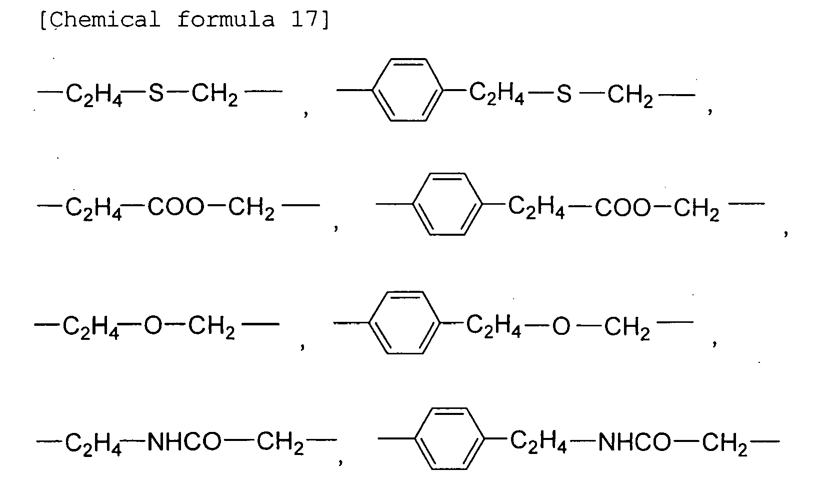 , etc.

In general formula (II), A is a C₁₋₂₄ divalent to octavalent organic group. The valence of A corresponds to the value of n of general formula (II). For example, when n is 2, A is a divalent group. When n is 3, A is a trivalent group.

Examples of divalent organic groups represented by A include those mentioned as examples of R.

Examples of trivalent to octavalent organic groups represented by A include aliphatic hydrocarbons, aromatic hydrocarbons, alicyclic hydrocarbons, and combinations thereof. Some or all of the hydrocarbon atoms bonded to the aromatic ring may be substituted with C₁₋₆ lower alkyl groups or halogen atoms. Some of the hydrogen atoms of the aliphatic hydrocarbon group may be substituted with halogen atom(s). The organic group may contain a heteroatom as described above for R.

Examples of said at least trivalent aromatic hydrocarbons include the following trivalent aromatic hydrocarbons: 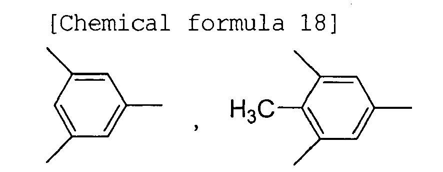 , etc.;
tetravalent aromatic hydrocarbons: 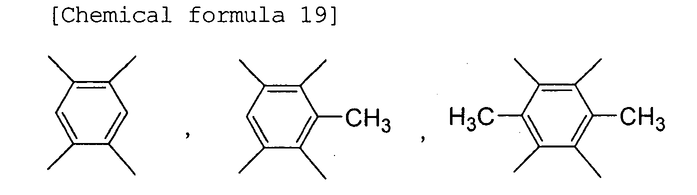 , etc.; and
pentavalent to octavalent aromatic hydrocarbons: 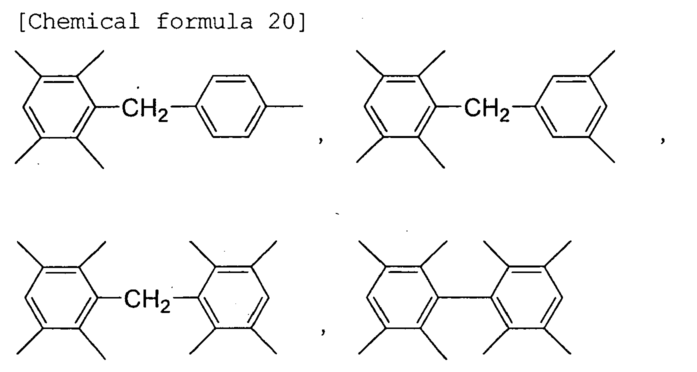 , etc.

In general formula (II), examples of trivalent to octavalent aliphatic hydrocarbons represented by A include the following: 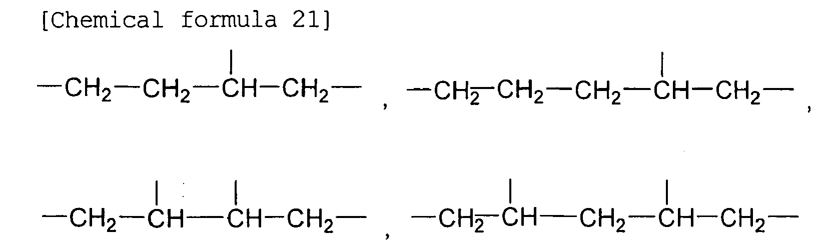 , etc.

In general formula (II), examples of trivalent to octavalent alicyclic hydrocarbons represented by A include the following: 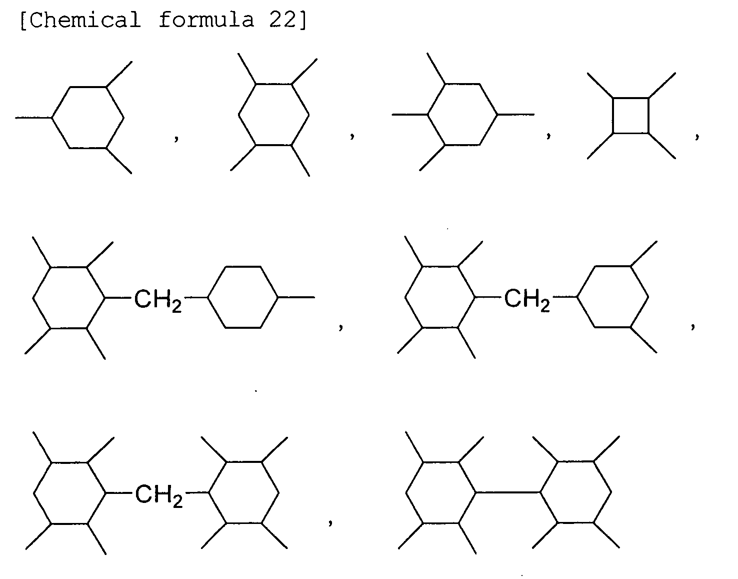 , etc.

Of the compounds of general formula (II), those in which R and A are as defined below are preferable in view of the excellent curability of compositions containing such compounds and the excellent thermal cleavability of tertiary ester bonds in cured products thereof. The group represented by R and the group represented by A can be suitably selected, in combination, from those mentioned below.

Compounds wherein m is 1, and R is a divalent group such as -CH₂-, -C₂H₄-, -C₃H₆-,-O-CH₂-, -CH₂-O-CH₂-, -C₂H₄-O-CH₂- or -C₃H₆-O-CH₂-. The left chain of the divalent group is bonded to the carbon of a tertiary ester bond, and the right chain is to the carbon of an olefin oxide.

Compounds wherein A is a divalent or trivalent group, including, for example, the following: 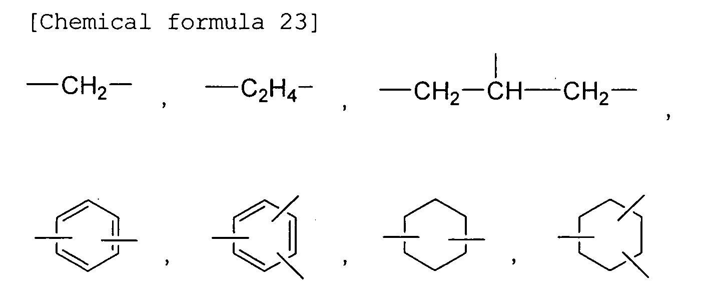 , etc.

Specific examples of preferable polyepoxy compounds of general formula (II) include the following:

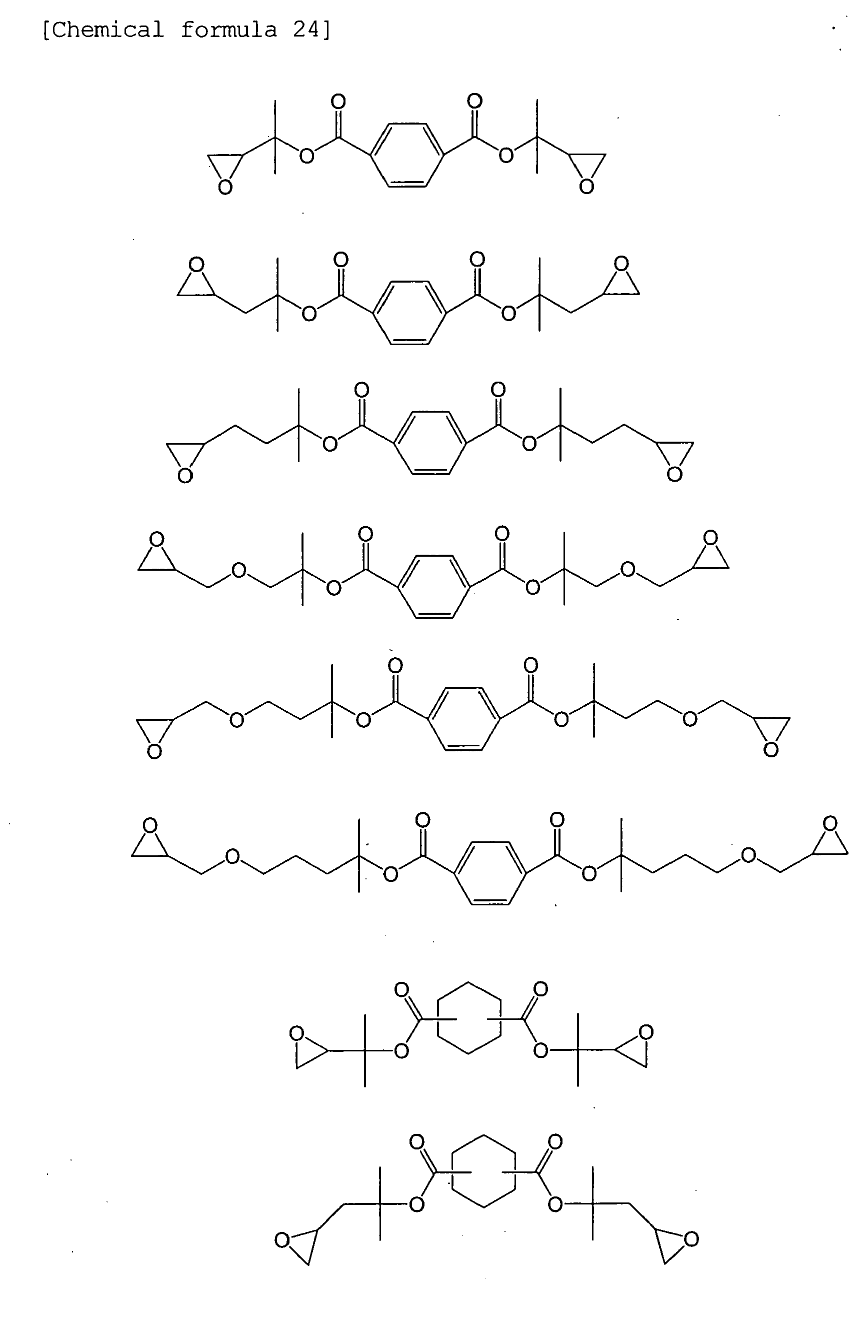

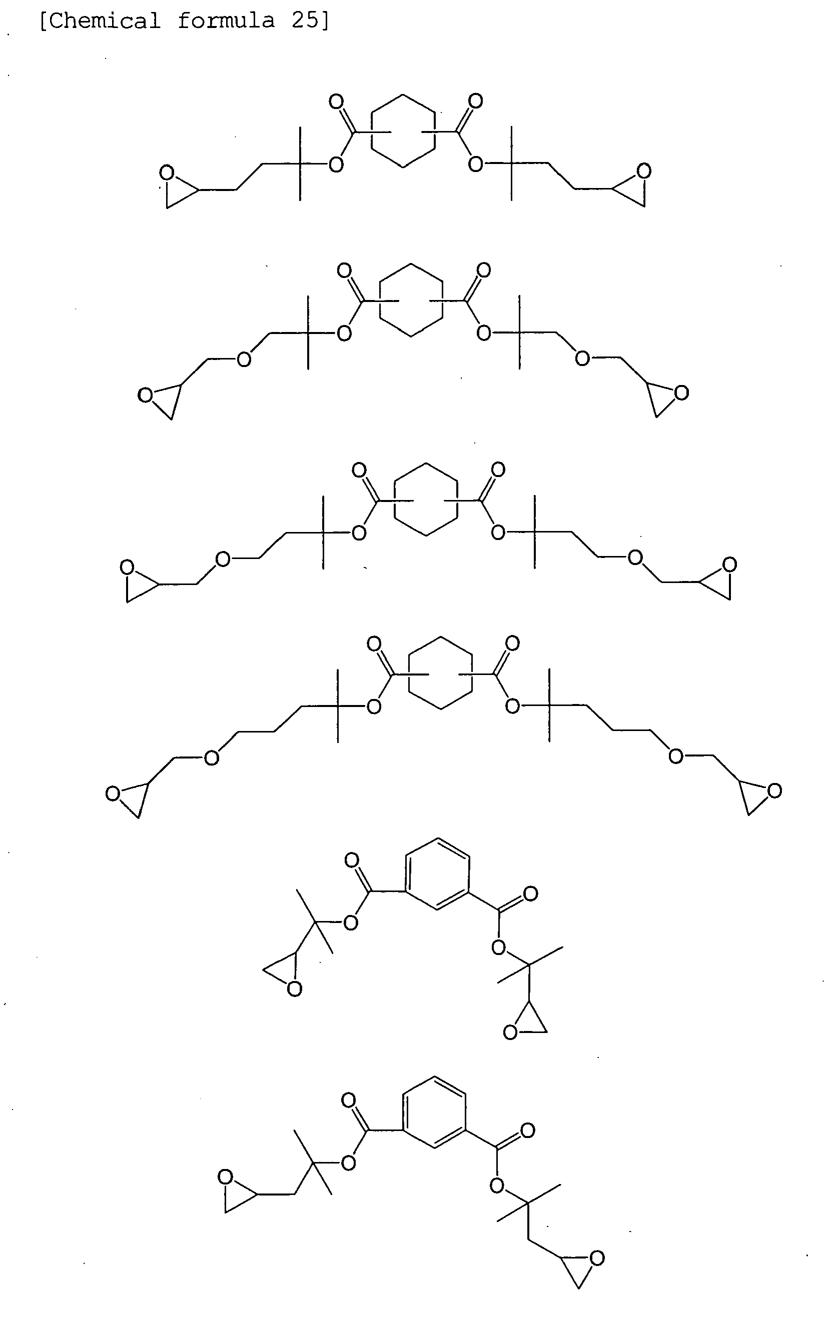

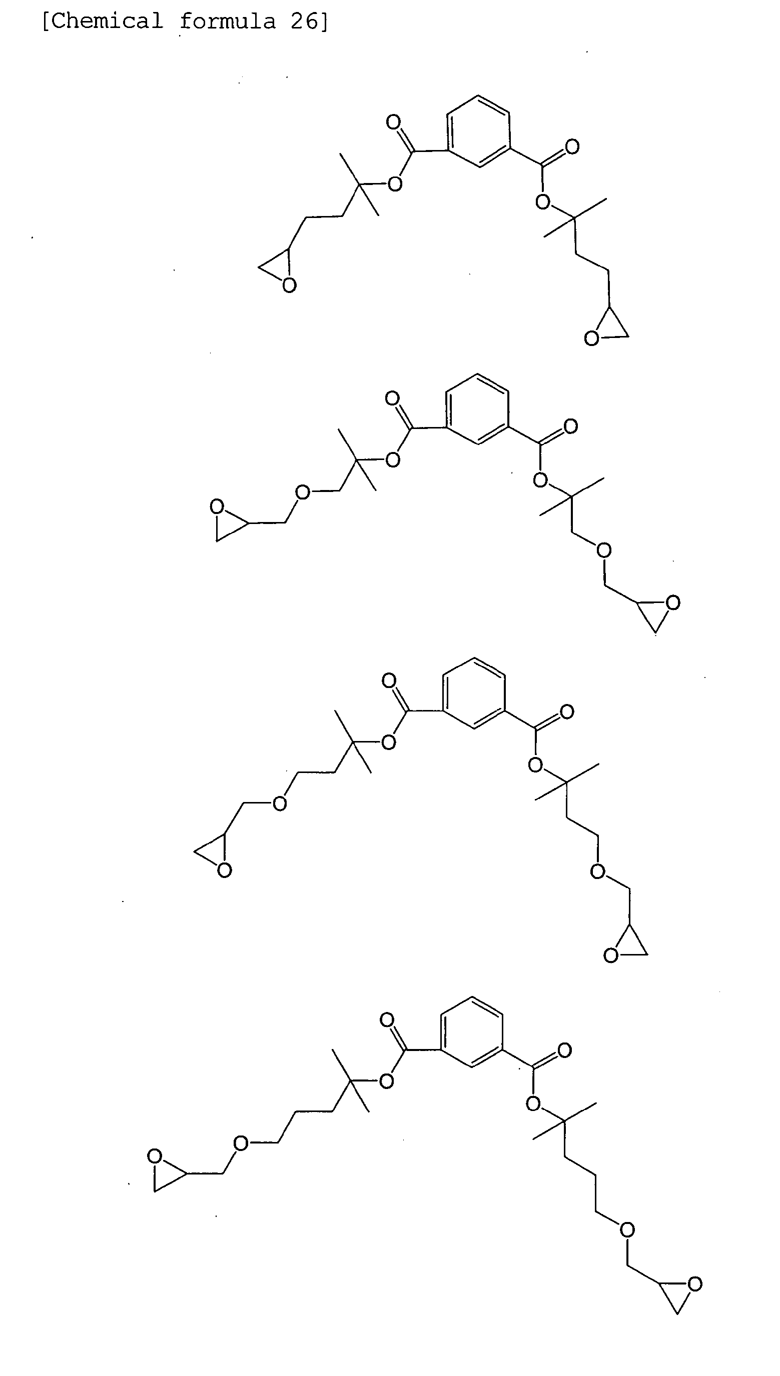

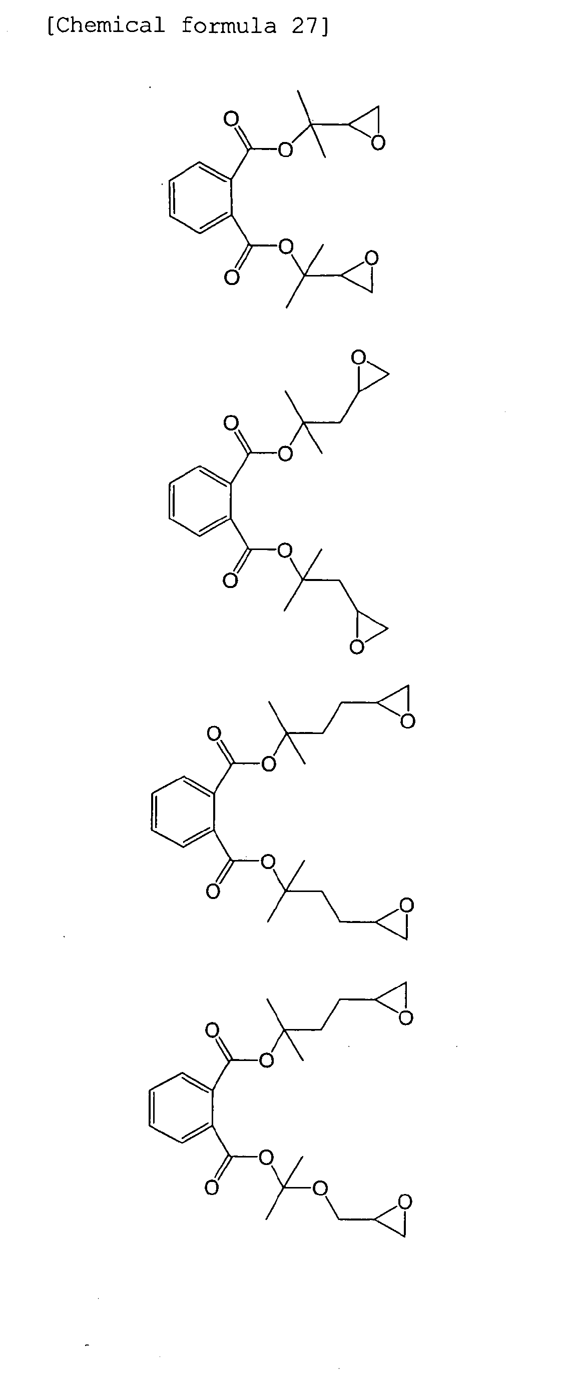

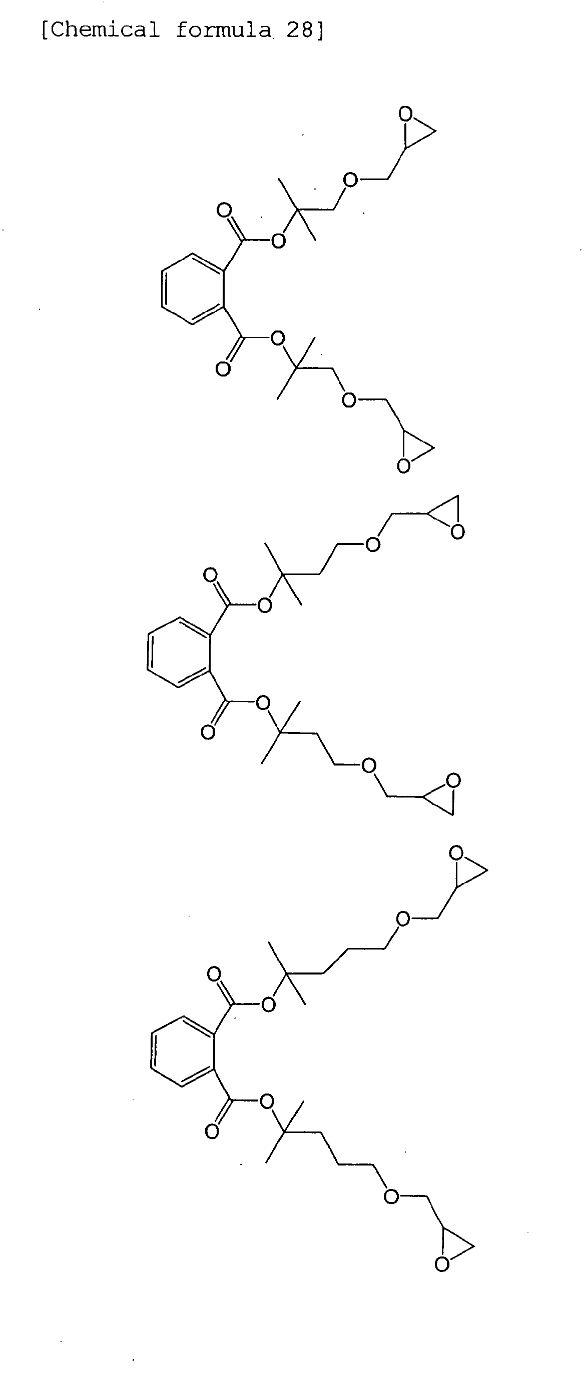

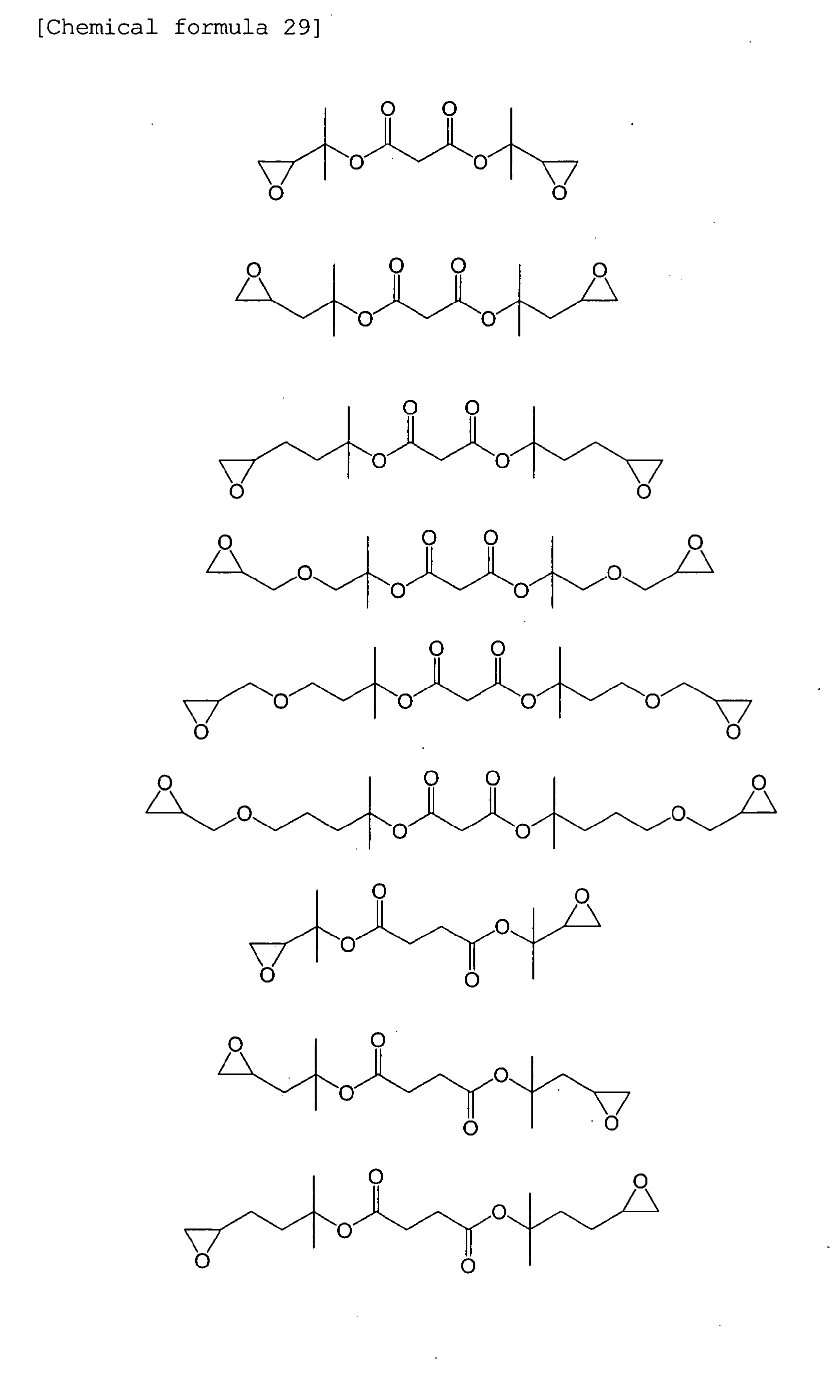

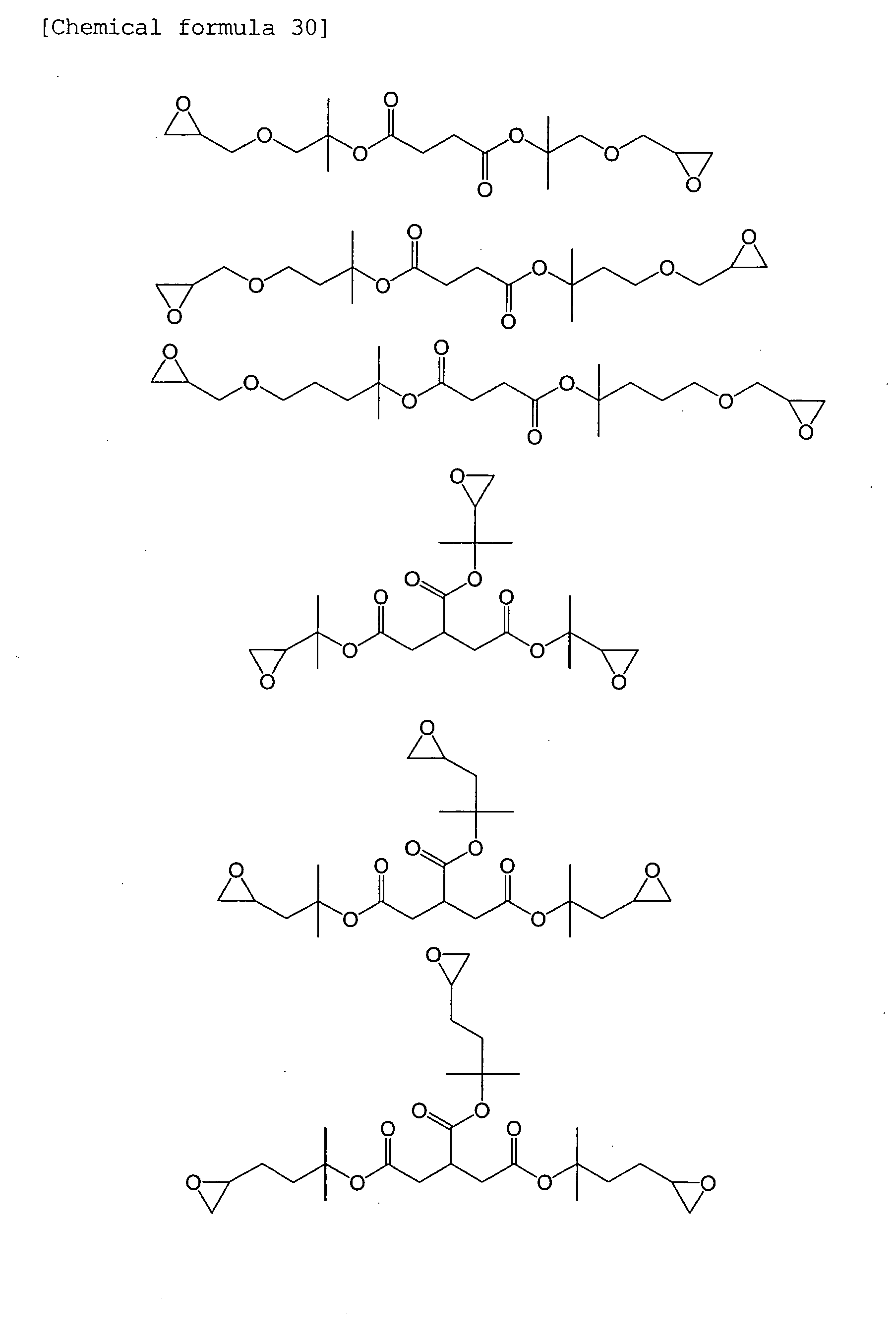

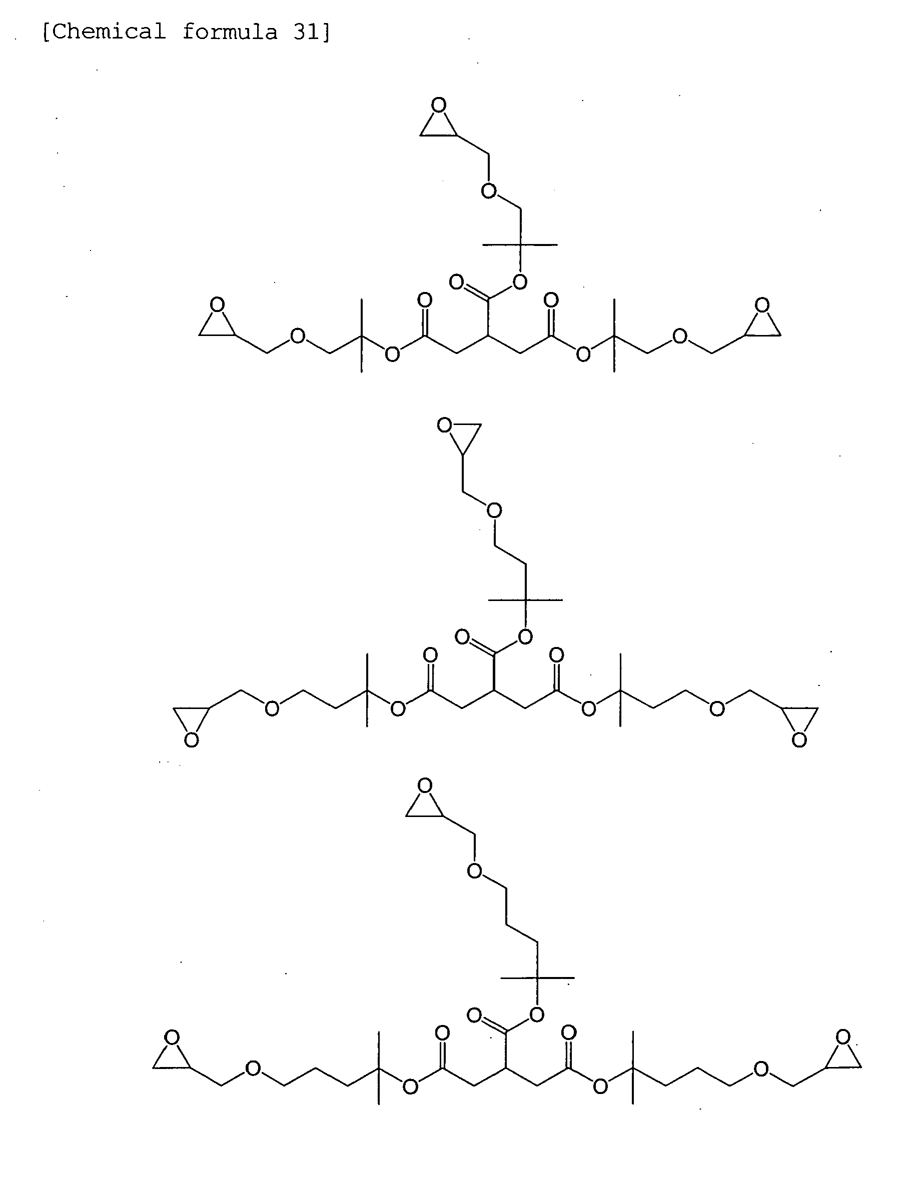

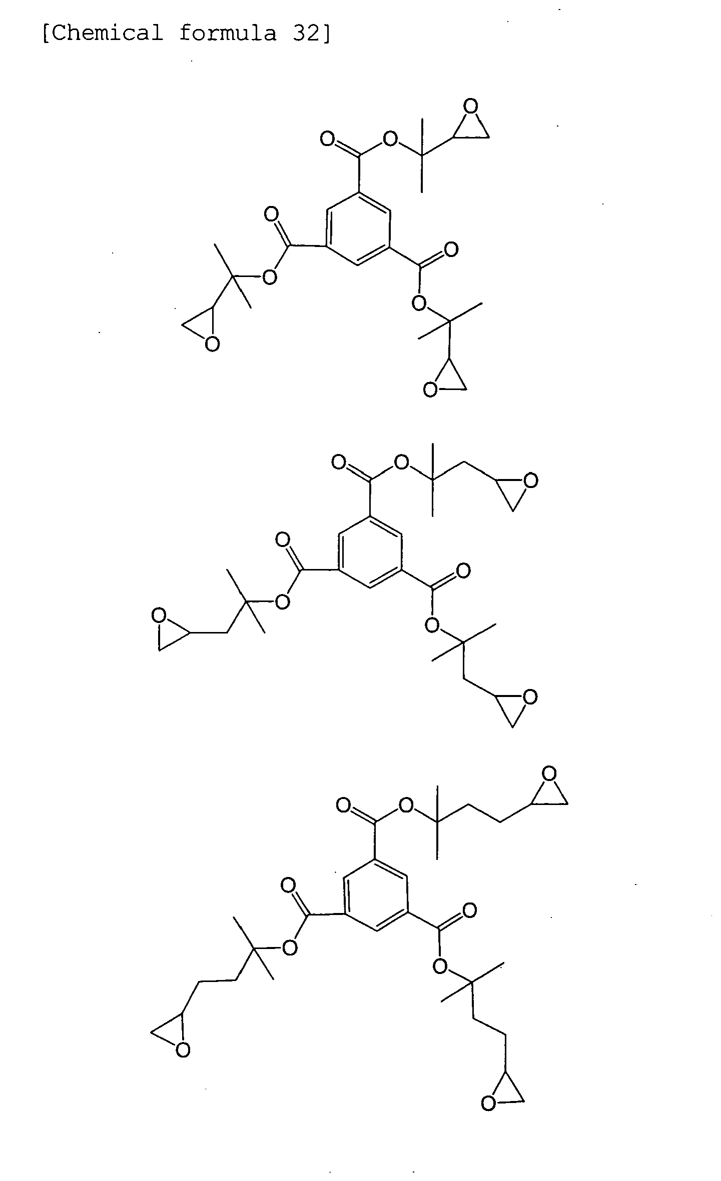

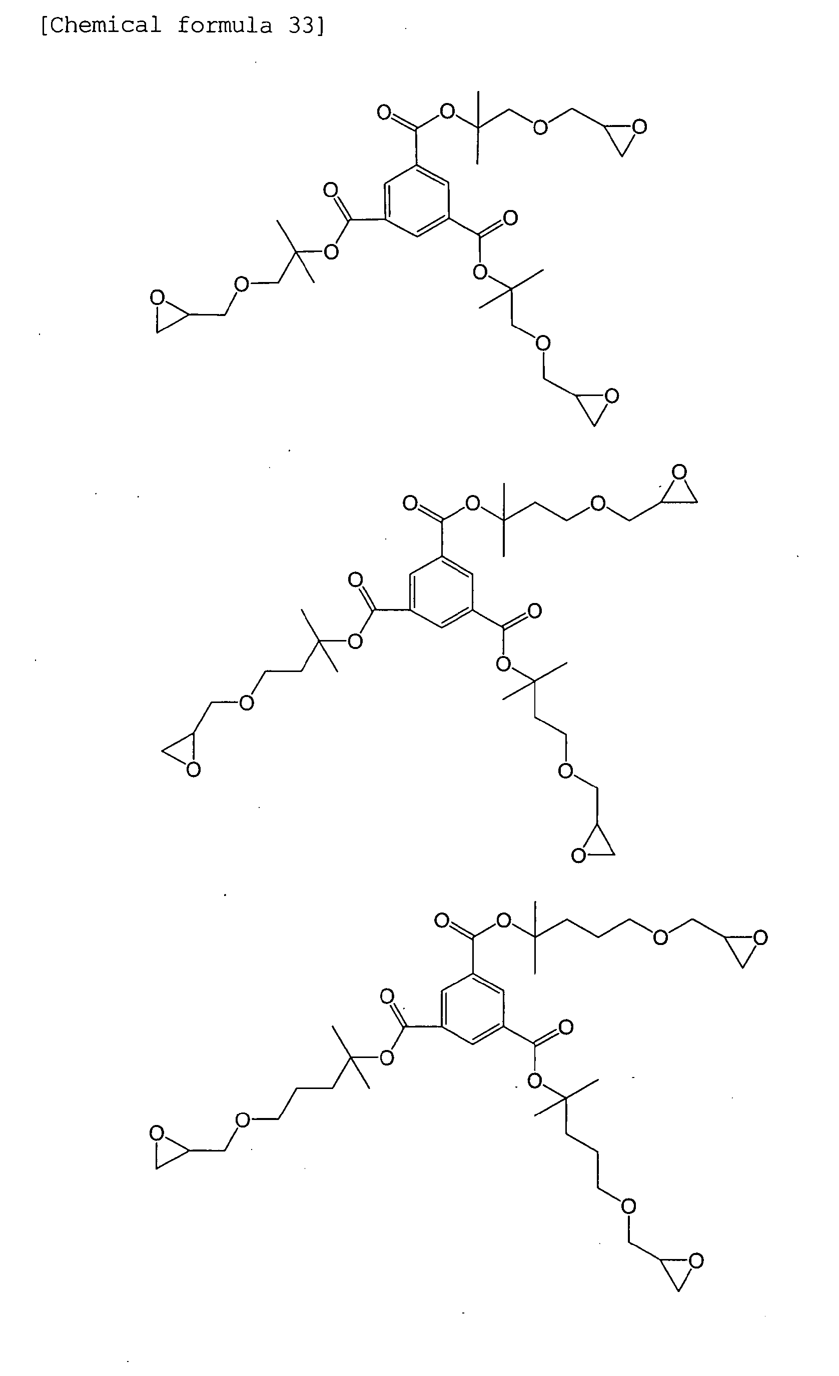

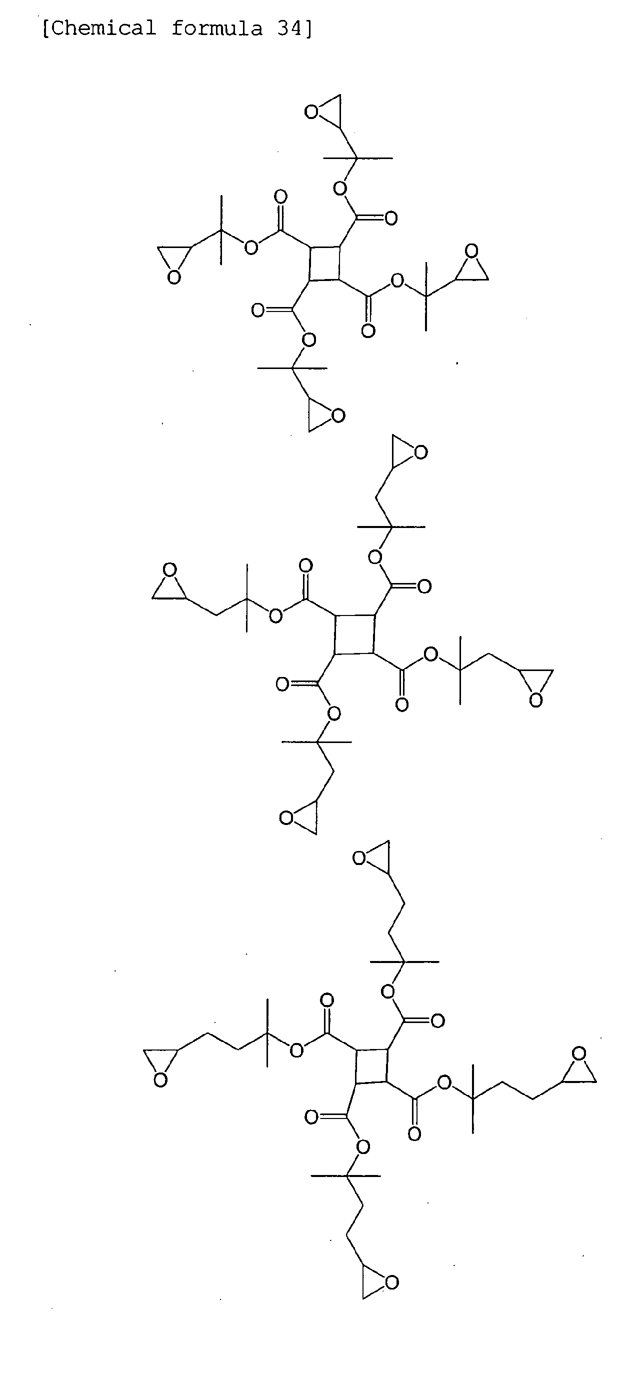

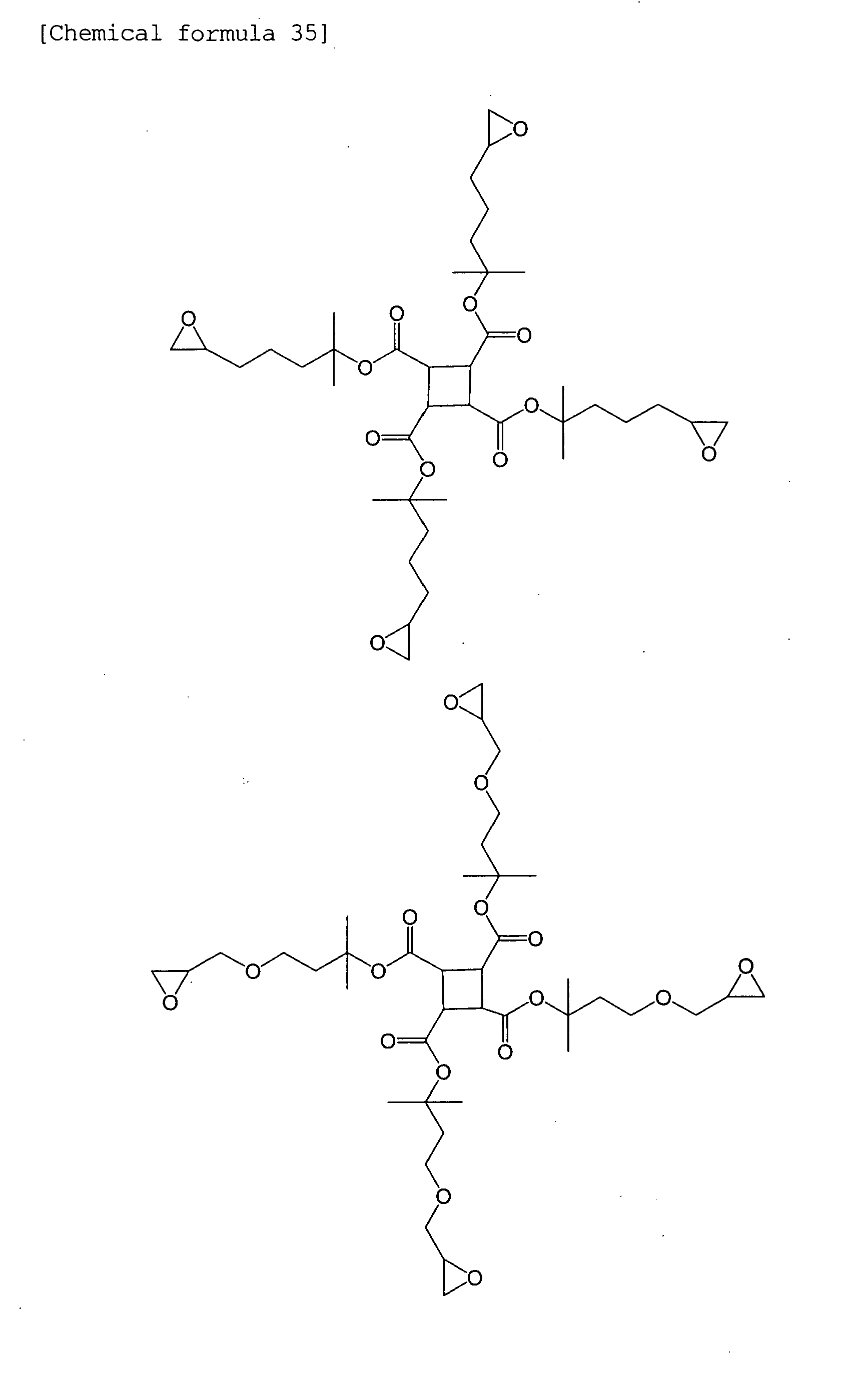

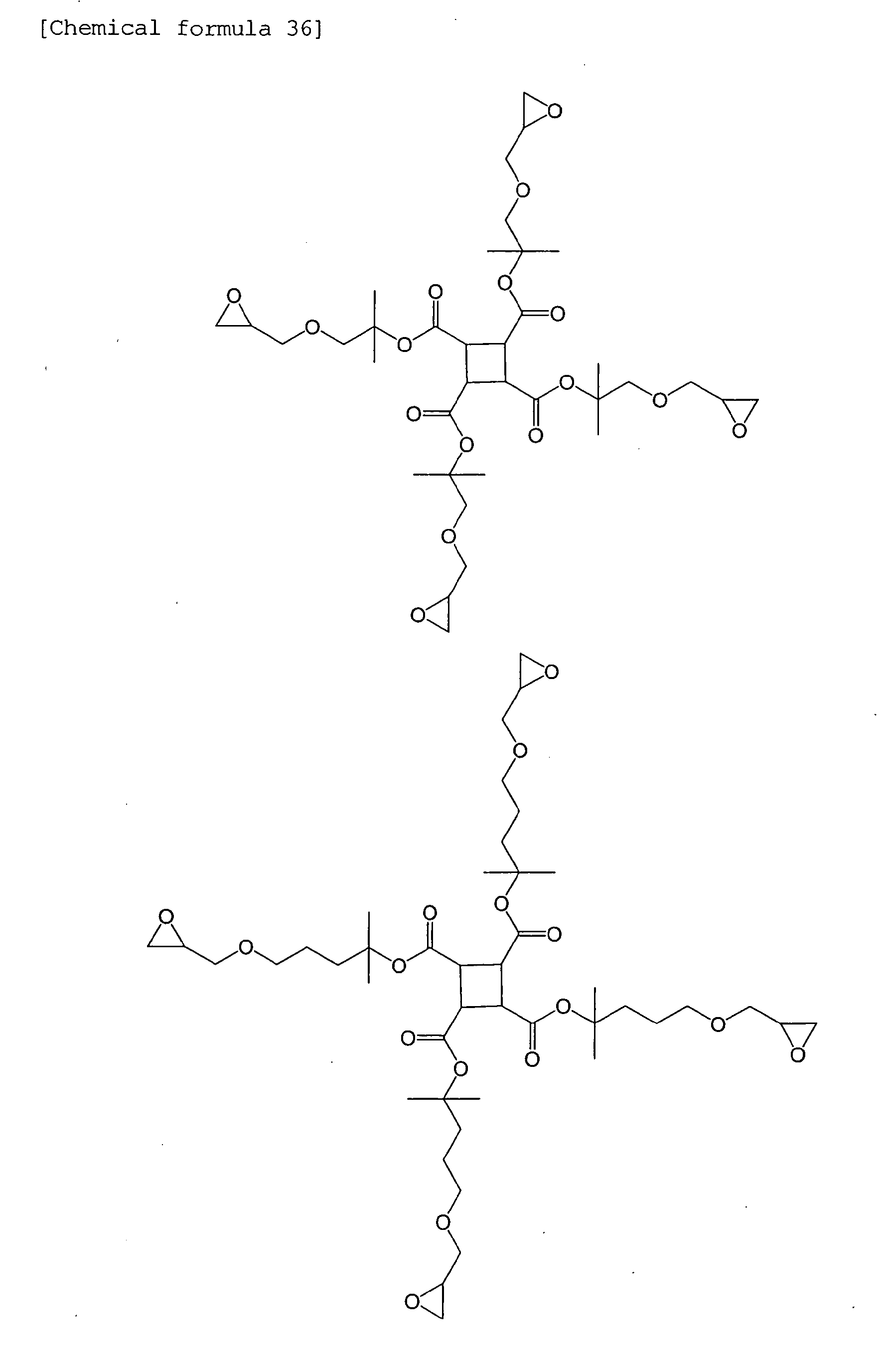

Specific examples of particularly preferable polyepoxy compounds of general formula (II) include compounds represented by the following formulas 1 to 5:

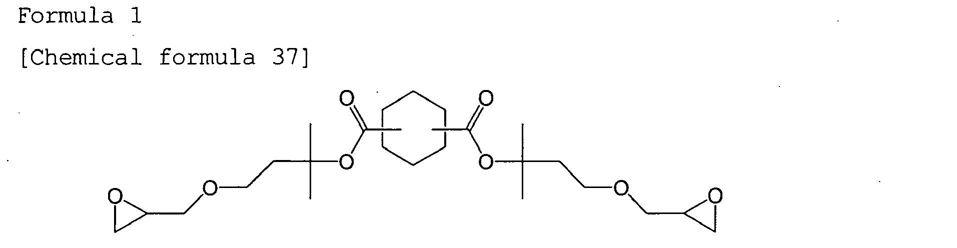

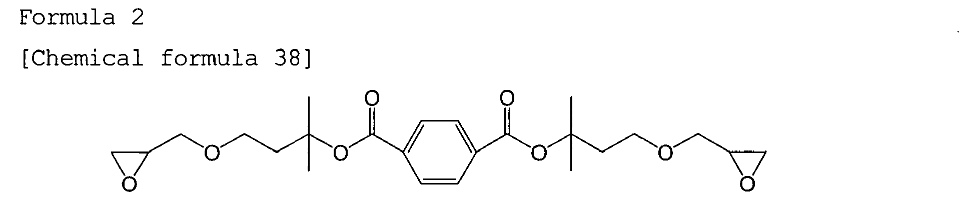

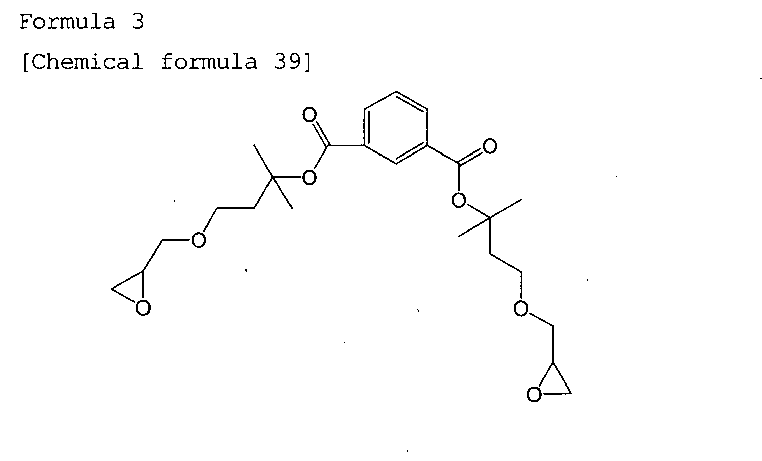

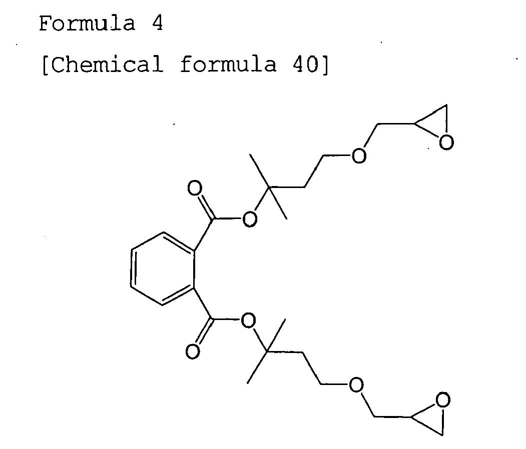

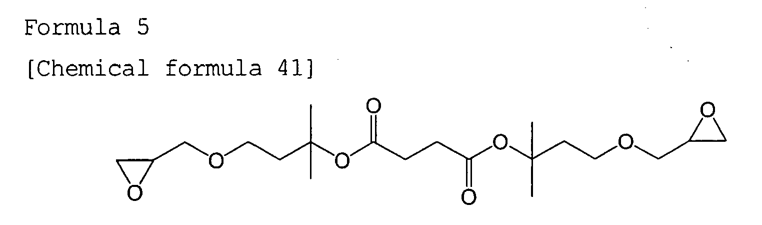

### Method of Producing the Polyepoxy Compound

A preferable method of producing the polyepoxy compound of the invention comprises esterifying at least one compound selected from the group consisting of:
(a) compounds containing per molecule at least two haloformyl groups represented by formula (III) 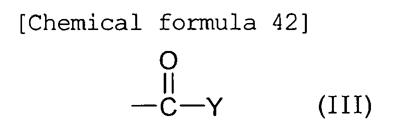 wherein Y is a halogen atom; (b) polycarboxylic acids; and
(c) acid anhydrides
   with an olefin bond-containing tertiary alcohol to produce an intermediate containing olefin bonds and tertiary ester bonds, and then oxidizing the olefin bonds of the intermediate to produce a compound containing olefin oxide groups and tertiary ester groups.

### Olefin bond-containing tertiary alcohol

Usable olefin bond-containing tertiary alcohols are not particularly limited as long as they have an olefin bond and a tertiary hydroxyl group per molecule. Known alcohols can be used.

The olefin bond-containing tertiary alcohol is preferably a compound represented by general formula (IV) 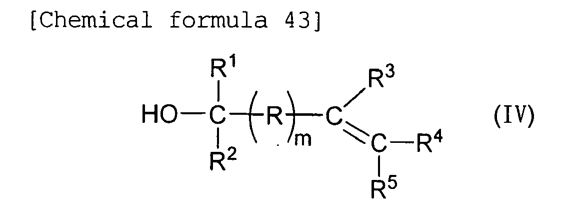 wherein R, R¹, R², R³, R⁴, R⁵ and m are as defined above.

Specific examples of olefin bond-containing tertiary alcohols include 2-methyl-3-buten-2-ol, 3-methyl-1-penten-3-ol, 3-methyl-5-hexen-3-ol, 3-allyloxy-2-methylpropan-2-ol, 4-4-allyloxy-2-methylbutan-2-ol, 5-allyloxy-2-methylheptan-2-ol, etc.

Examples of olefin bond-containing tertiary alcohols of general formula (IV) wherein R is an ether group-containing organic group include allylether compounds represented by general formula (V) 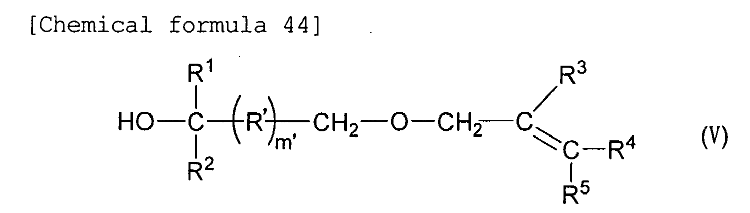 wherein m' is an integer of 0 or 1, R' is a C₁₋₂₂ divalent organic group, and R¹, R², R³, R⁴ and R⁵ are as defined above.

Such allylether compounds can be produced by reacting a primary hydroxyl- and tertiary hydroxyl-containing compound with a halogenated methallyl or halogenated allyl compound under basic conditions. After the reaction, the reaction product is extracted by a liquid-liquid extraction process using an aqueous solvent and an non-aqueous solvent to extract the unreacted raw materials in the aqueous solvent phase and extract the desired allylether compound in the non-aqueous solvent phase, thereby giving the desired compound with high purity and high yield.

The C₁₋₂₂ divalent organic group represented by R' refers to organic groups containing 1 to 22 carbon atoms of the category of C₁₋₂₄ divalent organic groups represented by R.

To produce such allylether compounds, the reaction ratio of the primary hydroxyl- and tertiary hydroxyl-containing compound to the halogenated methallyl or halogenated allyl compound is preferably such that 0.5 to 5 equivalents of the latter compound is used per equivalent of the primary hydroxyl of the former compound.

To establish basic conditions, metal hydroxides, metal hydrides, etc. can be used. Examples of metal hydroxides include hydroxides of alkali metals such as sodium, potassium, lithium, etc.; and hydroxides of alkali earth metals such as calcium, magnesium, etc. Examples of metal hydrides include hydrides of alkali metals such as sodium, potassium, lithium, etc.; and hydrides of alkaline earth metals such as calcium, magnesium, etc.; antimony hydride, silicon hydride, etc.

### Olefin bond- and tertiary ester-containing intermediate

The olefin bond- and tertiary ester-containing intermediate can be produced by esterifying said olefin bond-containing tertiary alcohol and at least one compound selected from the group consisting of (a) compounds containing at least 2 haloformyl groups of general formula (III), (b) polycarboxylic acids, and (c) acid anhydrides.

Examples of haloformyl group-containing compounds include those represented by general formula (VI) 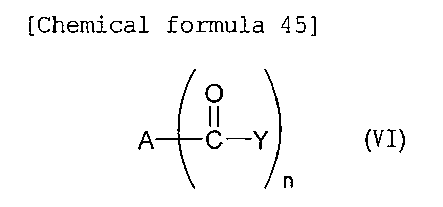 wherein A, n and Y are as defined above.

Of the haloformyl-containing compounds represented by general formula (VI), specific examples of aromatic compounds include terephthalic acid dichloride, isophthalic acid dichloride, orthophthalic acid dichloride, biphenyldicarboxylic acid dichloride, naphthalene dicarboxylic acid dichloride, trimesic acid trichloride, etc.

Of the haloformyl-containing compounds represented by general formula (VI), specific examples of aliphatic compounds include propane tricarboxylic acid trichloride, butane tricarboxylic acid trichloride, pentane tricarboxylic acid trichloride, malonic acid dichloride, succinic acid dichloride, glutaric acid dichloride, adipic acid dichloride, dimethylmalonic acid dichloride, etc.

Of the haloformyl-containing compounds represented by general formula (VI), specific examples of alicyclic compounds include cyclopropane tricarboxylic acid trichloride, cyclobutane tetracarboxylic acid tetrachloride, cyclopentane tricarboxylic acid trichloride, cyclopentane tetracarboxylic acid tetrachloride, cyclohexane tricarboxylic acid trichloride, cyclohexane tetracarboxylic acid tetrachloride, tetrahydrofuran tetracarboxylic acid tetrachloride, cyclopentane dicarboxylic acid dichloride, cyclobutane dicarboxylic acid dichloride, cyclohexane dicarboxylic acid dichloride, tetrahydrofuran dicarboxylic acid dichloride, etc.

Although only chlorides are mentioned above as examples of haloformyl-containing compounds of general formula (VI), bromides, iodides, and fluorides can also be used.

The esterification reaction can be carried out using an esterification catalyst. Examples of esterification catalysts include organic bases and inorganic bases. Specific examples include organic bases such as pyridine, dimethylaminopyridine, triethylamine, tripropylamine, N,N-diisopropylethylamine, tributylamine, trioctylamine, N-methylpiperidine, N-ethylpiperazine, N-methylmorpholine, N-ethylmorpholine, N,N-dimethylaniline, N,N-diethylaniline; and inorganic bases such as sodium hydroxide, potassium hydroxide, sodium carbonate, sodium hydrogencarbonate, potassium carbonate, potassium hydrogencarbonate, etc.

The esterification reaction can be carried out using an organic solvent. Specific examples thereof include linear paraffins such as pentane, hexane, heptane, octane, nonane, decane, undecane, dodecane, tridecane, tetradecane, pentadecane, hexadecane, heptadecane, octadecane, etc.; branched paraffins obtained by substitution of such linear paraffins with alkyl such as methyl, dimethyl, trimethyl, ethyl, diethyl, triethyl, propyl, dipropyl, tripropyl, butyl, etc.; olefins that are monoenes, dienes and trienes of such linear or branched paraffins; cycloparaffins such as cyclopentane, cyclohexane, cycloheptane, cyclooctane, cyclononane, cyclodecane, dicyclopropylmethane, dicyclopentyl, dicyclohexyl, norbornene, etc.; branched cycloparaffins obtained by substitution of such cycloparaffins with alkyl such as methyl, dimethyl, trimethyl, ethyl, diethyl, triethyl, propyl, dipropyl, tripropyl, butyl, etc.; olefins that are monoenes, dienes and trienes of such cycloparaffins or branched cycloparaffins; aromatic hydrocarbons such as benzene, toluene, ethylbenzene, propylbenzene, butylbenzene, pentylbenzene, phenylhexane, phenylheptane, phenyloctane, phenylnonane, phenyldecane, phenylundecane, phenyldodecane, cyclopropylphenylmethane, cyclohexylbenzene, diphenylmethane, xylene, ethyltoluene, diethylbenzene, dipropylbenzene, dibutylbenzene, tolylmethane, tolylethane, trimethylbenzene, tetramethylbenzene, triethylbenzene, tripropyl benzene, etc.; and halogen solvents such as chloroform, methylene chloride, etc. Such organic solvents can be used singly or in a combination of two or more. Further examples of usable solvents include commercially available industrial solvents such as petroleum ether, petroleum benzine, gasolines, etc.

The olefin bond- and tertiary ester-containing intermediate can be produced by dissolving an olefin bond-containing tertiary alcohol and a haloformyl-containing compound in an organic solvent, then adding dropwise thereto such an organic solvent solution of an esterification catalyst as mentioned above and allowing the reaction to proceed.

The reaction ratio of the olefin bond-containing tertiary alcohol to the haloformyl-containing compound is preferably such that about 2 to about 20 moles, and more preferably about 2 to about 10 moles, of the former tertiary alcohol is used per mole of the latter compound. The amount of organic solvent is preferably such that the total concentration of the olefin bond-containing tertiary alcohol and haloformyl-containing compound becomes about 5 to about 80 wt.%, and more preferably about 10 to about 50 wt.%. When the amount of olefin bond-containing tertiary alcohol compound is less than 2 moles or the total concentration of the tertiary alcohol compound and haloformyl-containing compound is not within the range of 5 to 80 wt.%, the yield and reaction efficiency tend to be reduced.

The amount of esterification catalyst is preferably about 0.005 to about 5 moles, more preferably about 0.01 to about 3 moles per mole of the haloformyl-containing compound. The organic solvent solution of an esterification catalyst preferably has an esterification catalyst concentration of about 1 to about 95 wt.%, and more preferably about 2 to about 50 wt.%. When the amount of esterification catalyst is less than 0.005 moles or the esterification catalyst concentration is less than 1 wt.%, the yield and reaction efficiency tend to be reduced.

The reaction temperature is preferably about -78°C to about 150°C, and more preferably about 0°C to about 100°C. The organic solvent solution of an esterification catalyst is preferably added dropwise with stirring over a period of about 0.1 to about 3 hours, and more preferably about 0.2 to about 1.5 hours. When the reaction temperature is less than -78°C, the reaction requires more time and reduced reaction efficiency tends to result. When the reaction temperature is more than 150°C, the olefin bond-containing tertiary alcohol compound starts to polymerize and tends to result in a low yield. When the esterification catalyst solution is added over a period of less than 0.1 hour, the reaction becomes too intense and results in difficulty in controlling the temperature, whereby a low yield tends to result. When the esterification catalyst solution is added dropwise over a period of more than 3 hours, the yield does not increase. After the dropwise addition, the mixture is further stirred to mature. It is usually preferable that the maturation period be 1 month or less, and more preferably not more than 3 weeks.

The haloformyl-containing compound and the olefin bond-containing tertiary alcohol compound can be reacted in the presence of an esterification catalyst, for example, by the following methods:
(i) A method as mentioned above, which comprises adding an organic solvent solution of an esterification catalyst dropwise to an organic solvent solution of a haloformyl-containing compound and an olefin bond-containing tertiary alcohol compound and allowing the reaction to proceed.
(ii) A method comprising adding an organic solvent solution of an olefin bond-containing tertiary alcohol and an esterification catalyst dropwise to a solution of a haloformyl-containing compound and allowing the reaction to proceed.
(iii) A method comprising adding an organic solvent solution of a haloformyl-containing compound and an olefin bond-containing tertiary alcohol dropwise to an organic solvent solution of an esterification catalyst and allowing the reaction to proceed.
(iv) A method comprising adding an organic solvent solution of a haloformyl-containing compound to an organic solvent solution of an esterification catalyst and an olefin bond-containing tertiary alcohol and allowing the reaction to proceed.

Compounds containing at least 2 carboxyl groups per molecule can be used as polycarboxylic acid (b). Specific examples include aromatic dicarboxylic acids such as terephthalic acid, isophthalic acid, orthophthalic acid, 2,6-naphthalenedicarboxylic acid, biphenyldicarboxylic acid, etc.; aliphatic dicarboxylic acids such as succinic acid, adipic acid, azelaic acid, sebacic acid, dodecane diacid, dimer acid, 1,4-cyclohexanedicarboxylic acid, etc.; at least trifunctional (or tri- or poly-functional) aromatic polycarboxylic acids such as trimellitic acid, pyromellitic acid, benzophenonetetracarboxylic acid, etc.; and at least trifunctional (or tri- or polyfunctional) aliphatic polycarboxylic acids such as butane tetracarboxylic acid, etc.

Compounds containing at least 1 acid anhydride per molecule can be used as acid anhydride (c). Specific examples include maleic anhydride, dodecenylsuccinic anhydride, chlorendic anhydride, sebacic anhydride polymer, phthalic anhydride, trimellitic anhydride, pyromellitic anhydride, cyclopentane tetracarboxylic dianhydride, hexahydrophthalic anhydride, endomethylenehexahydrophthalic anhydride, methylnasic anhydride, benzophenonetetracarboxylic dianhydride, etc.

The polycarboxylic acid (b) can produce an intermediate containing olefin bonds and tertiary ester bonds by esterification of its carboxyl groups with tertiary hydroxyl groups of an olefin bond-containing tertiary alcohol. The acid anhydride (c) can produce an intermediate containing olefin bonds and tertiary ester bonds by an addition reaction of its acid anhydride groups with tertiary hydroxyl groups of an olefin bond-containing tertiary alcohol.

The reaction of the olefin bond-containing tertiary alcohol compound with the polycarboxylic acid or acid anhydride can be carried out by a known esterification reaction. If necessary, an esterification catalyst as mentioned above can be used to perform the reaction. The reaction ratio of the olefin bond-containing tertiary alcohol to the polycarboxylic acid or acid anhydride is preferably about 2 to about 20 moles, and more preferably about 2 to about 10 moles, of the olefin bond-containing tertiary alcohol used per mole of the polycarboxylic acid or acid anhydride.

After the reaction of the olefin bond-containing tertiary alcohol with at least one compound selected from the group consisting of haloformyl-containing compounds (a), polycarboxylic acids (b), and acid anhydrides (c), purification can be performed by known methods such as washing with water, distillation, silica gel column chromatography, etc. Specific examples of purification methods include a method comprising washing with water to remove hydrochloride and then subjecting to distillation to purify the desired product; a method comprising directly performing distillation without washing with water; a method consisting of washing with water and distilling off the solvent; etc. Since the obtained compound contains a tertiary ester bond which is easily cleaved by heating, performing purification using a thin-film distillation apparatus that enables distillation while reducing heat history and thereby suppressing thermal cleavage is effective.

Thin-film distillation can be performed using known apparatus and methods. The thin-film distillation is preferably performed under the following conditions: a temperature of about 100°C to about 200°C and a pressure of about 0.01 torr to about 10 torr. If necessary, the thin-film distillation can be performed more than once.

### Oxidation of the olefin bond- and tertiary ester-containing intermediate

A final product polyepoxy compound of the invention can be obtained by oxidation (epoxidation) of the olefin bonds of the intermediate to form olefin oxide groups such as glycidyl, methylglycidyl, etc.

The oxidation (epoxidation) can be performed by dissolving the intermediate in an organic solvent and then adding a peroxide oxidizing agent at a temperature of about 0°C to about 40°C to allow the reaction to proceed at about room temperature for about 8 to about 24 hours.

Examples of usable peroxide oxidizing agents include m-chloroperbenzoic acid, peracetic acid, hydrogen peroxide, etc. The amount of oxidizing agent, when it provides one oxygen atom, is preferably about 1.5 to about 20 moles, and more preferably about 2 to 5 moles, per mole of the intermediate.

Preferable examples of such organic solvents include halogen solvents such as methylene chloride, etc.; and aromatic hydrocarbon solvents such as hexane, toluene, etc. Methylene chloride is particularly preferable.

It is also possible to use dimethyldioxisilane as an oxidizing agent to epoxidate the olefin bond (see Chem. Mater. 1998, 10, 3724).

The polyepoxy compound of the invention can be used, for example, as an epoxy resin component in coating compositions, ink compositions, adhesive compositions, molded products, composite materials, etc. Examples of molded products include automobile exterior parts, electronic parts, etc.

When the epoxy compound of the invention is used, for example, for the above purposes as an epoxy resin component and the obtained composition is cured by heating, a tertiary ester bond derived from the epoxy compound in the cured product is thermally cleaved by post-heating the cured product at a temperature higher than the curing temperature, so that the crosslinking structure is cleaved, thus enabling removal of the cured product.

The polyepoxy compound of the invention is useful as a component of a curing composition further containing a resin with a functional group capable of reacting with an epoxy group, a curing agent capable of reacting with an epoxy group, etc. The epoxy compound of the invention is particularly preferable for use as an epoxy resin component of thermosetting resin compositions shown below in (1) and (2):
(1) thermosetting resin composition I comprising a carboxyl- and/or hydroxyphenyl-containing resin and an epoxy compound of the invention; and
(2) thermosetting resin composition II containing a polyepoxy compound of the invention and an epoxy curing agent.

These thermosetting resin compositions are described below.

### Thermosetting resin composition I

The thermosetting resin composition I of the invention contains a carboxyl- and/or hydroxyphenyl-containing resin (A) and the above polyepoxy compound (B) of the invention.

### Carboxyl- and/or hydroxyphenyl-containing resin (A)

Preferable examples of carboxyl- and/or hydroxyphenyl-containing resins (A) include carboxyl-containing resins (a) having a weight average molecular weight of about 500 to about 100,000, a glass transition point of about 0°C to about 200°C, and an acid value of about 5 to about 700 mgKOH/g; hydroxyphenyl-containing resins (b) having a weight average molecular weight of about 500 to about 100,000, a glass transition point of about 0°C to about 200°C, and a hydroxyl value of about 5 to about 600 mgKOH/g; and carboxyl- and hydroxyphenyl-containing resins (c) having a weight average molecular weight of about 500 to about 100,000, an acid value of about 5 to about 700 mgKOH/g, a glass transition point of about 0°C to about 200°C, and a hydroxyl value of about 5 to about 600 mgKOH/g.

### Carboxyl-containing resin (a)

Resin (a) contains carboxyl group(s) and is capable of forming a coating film. Examples of compounds usable as resin (a) include homopolymers of carboxyl-containing polymerizable unsaturated monomers; copolymers of a carboxyl-containing polymerizable unsaturated monomer and other copolymerizable unsaturated monomer(s); carboxyl-containing polyester resins, carboxyl-containing polyurethane resins, etc.

Such a homopolymer of a carboxyl-containing polymerizable unsaturated monomer, and such a copolymer of a carboxyl-containing polymerizable unsaturated monomer and other unsaturated monomer(s) copolymerizable therewith can be prepared by subjecting the respective specified monomers to a known radical polymerization reaction.

Examples of carboxyl-containing polymerizable unsaturated monomers include acrylic acid, methacrylic acid, crotonic acid, itaconic acid, etc. Examples of other monomers copolymerizable with such carboxyl-containing polymerizable unsaturated monomers include C₁₋₁₂ alkyl esters of (meth)acrylic acid, such as methyl (meth)acrylate, ethyl (meth)acrylate, propyl (meth)acrylate, butyl (meth)acrylate, hexyl (meth)acrylate, octyl (meth)acrylate, and 2-ethylhexyl (meth)acrylate, nonyl (meth)acrylate, decyl (meth)acrylate, etc.; C₂₋₆ hydroxyalkyl esters of (meth)acrylic acid, such as hydroxyethyl (meth)acrylate, 2-hydroxypropyl (meth)acrylate, 3-hydroxypropyl (meth)acrylate, hydroxybutyl (meth)acrylate, etc.; vinyl aromatic compounds such as styrene, α-methylstyrene, p-tert-butylstyrene, etc.; vinyl acetate, (meth)acrylonitrile, (meth)acrylamide, vinyl pyrrolidone, etc. Such monomers can be used singly or in a combination of two or more.

The carboxyl-containing polyester resin contains carboxyl group(s) in the molecular chain or at the molecular end(s), and can be prepared, for example, using a polybasic acid and a polyhydric alcohol as the main materials by a known esterification reaction.

Examples of usable polybasic acids include dicarboxylic acids and anhydrides thereof, such as terephthalic acid, isophthalic acid, phthalic anhydride, phthalic acid, 1,4-naphthol acid, diphenic acid, 4,4'-oxybenzoic acid, diglycolic acid, 2,5-naphthalenedicarboxylic acid, tetrahydrophthalic acid, hexahydrophthalic acid, tetrahydrophthalic anhydride, hexahydrophthalic anhydride, 4-methylhexahydrophthalic anhydride, tetrabromophthalic anhydride, tetrachlorophthalic anhydride, 2,5-norbornenedicarboxylic acid, oxalic acid, malonic acid, dimethylmalonic acid, succinic acid, glutaric acid, 2,2-dimethylglutaric acid, oxalic acid, adipic acid, dodecanedicarboxylic acid, azelaic acid, sebacic acid, 1,3-cyclohexyldicarboxylic acid, etc.; alkyl esters of such dicarboxylic acids containing about 1 to about 4 carbon atoms; etc. Such acids can be used singly or in a combination of two or more. Other examples include tri- or poly-functional carboxylic acids, such as trimellitic acid, trimellitic anhydride, pyromellitic acid, pyromellitic anhydride, butanetetracarboxylic acid, etc. Further, unsaturated dicarboxylic acids such as maleic anhydride, maleic acid, itaconic anhydride, fumaric acid, and esters thereof may be used in small amounts.

Examples of polyhydric alcohols include diols such as ethylene glycol, diethylene glycol, triethylene glycol, polyethylene glycol, 1,2-propylene glycol, 1,3-propylene glycol, dipropylene glycol, polypropylene glycol, neopentyl glycol, 1,5-pentanediol, 1,6-hexandiol, 2-ethyl-1,3-hexandiol, 2,2,4-trimethyl-1,3-pentanediol, 2-butyl-2-ethyl-1,3-propanediol, 1,4-cyclohexanedimethanol, polytetramethylene glycol, bisphenol A, hydrogenated bisphenol A, etc. Such polyhydric alcohols can be used singly or in a combination of two or more. Other examples of usable polyhydric alcohols include tri- or poly-functional alcohols such as trimethylolethane, trimethylolpropane, glycerol, pentaerythritol, dipentaerythritol, etc. Further examples of usable polyhydric alcohols include methylolbutanoic acid, dimethylol propionic acid, tricyclodecane dimethanol, etc.

In addition to such a polybasic acid and polyhydric alcohol, a monobasic acid such as t-butylbenzoic acid, benzoic acid, etc. may be used as a molecular weight modifier.

The carboxyl-containing polyurethane resin contains carboxyl group(s) in the molecular chain or at the molecular end(s), and can be prepared, for example, by subjecting a polyisocyanate compound, a carboxyl-containing polyol, and a polyol other than such a carboxyl-containing polyol to a known urethanization reaction.

Examples of such polyisocyanate compounds include diisocyanate compounds, for example, aliphatic diisocyanate compounds such as hexamethylene diisocyanate, trimethylene diisocyanate, 1,4-tetramethylene diisocyanate, pentamethylene diisocyanate, 1,2-propylene diisocyanate, 1,2-butylene diisocyanate, trimethyl hexamethylene diisocyanate, dimer acid diisocyanate, lysine diisocyanate, 2,3-butylene diisocyanate, 1,3-butylene diisocyanate, etc.; alicyclic diisocyanate compounds such as isophorone diisocyanate, 4,4'-methylenebis(cyclohexylisocyanate), methylcyclohexane-2,4-diisocyanate, methylcyclohexane-2,6-diisocyanate, 1,3-di(isocyanatomethyl)cyclohexane, 1,4-di(isocyanatomethyl)cyclohexane, 1,4-cyclohexane diisocyanate, 1,3-cyclopentane diisocyanate, 1,2-cyclohexane diisocyanate, etc.; and aromatic diisocyanate compounds such as xylylene diisocyanate, m-xylylene diisocyanate, tetramethylxylylene diisocyanate, tolylene diisocyanate, 4,4'-diphenylmethane diisocyanate, 1,5-naphthalene diisocyanate, 1,4-naphthalene diisocyanate, 4,4'-toluidine diisocyanate, 4,4'-diphenylether diisocyanate, m-phenylene diisocyanate, p-phenylene diisocyanate, 4,4'-biphenylene diisocyanate, 3,3'-dimethyl-4,4'-biphenylene diisocyanate, bis(4-isocyanatophenyl)sulfone, isopropylidenebis(4-phenylisocyanate), etc.; and polyisocyanate compounds containing three or more isocyanate groups, for example, triphenylmethane-4,4',4''-triisocyanate, 1,3,5-triisocyanato benzene, 2,4,6-triisocyanate toluene, 4,4'-dimethyldiphenylmethane-2,2',5,5'-tetraisocyanate, etc. Such compounds can be used singly or in a combination of two or more.

Examples of such carboxyl-containing polyols include dimethylolpropionic acid, dimethylolbutanoic acid, dimethylolvaleric acid, tartaric acid, 1,2-hydroxystearic acid, p-hydroxybenzoic acid, salicylic acid, malic acid, lactic acid, hydroxyacetic acid, 2,2-dimethyl-3-hydroxypropionic acid, etc. Such polyols can be used singly or in a combination of two or more.

Examples of polyols other than carboxyl-containing polyols include glycols such as ethylene glycol, propylene glycol, polyethylene glycol with a molecular weight of 6,000 or less, polypropylene glycol with a molecular weight of 6,000 or less, tetramethylene glycol, 1,4-butanediol, 1,3-butanediol, 2,3-butanediol, 1,2-butanediol, 3-methyl-1,2-butanediol, 1,2-pentanediol, 1,5-pentanediol, 1,4-pentanediol, 2,4-pentanediol, 2,3-dimethyltrimethylene glycol, tetramethylene glycol, 3-methyl-4,3-pentanediol, 3-methyl-4,5-pentanediol, 2,2,4-trimethyl-1,3-pentanediol, 1,6-hexandiol, 1,5-hexandiol, 1,4-hexandiol, 2,5-hexandiol, 1,4-cyclohexanedimethanol, neopentyl glycol, hydroxypivalic acid neopentyl glycol ester, 1,4-cyclohexanedimethanol, tricyclodecane dimethanol, polycaprolactone, hydrogenated bisphenol A, hydrogenated bisphenol F, alkylene oxide addition products of hydrogenated bisphenol A, alkylene oxide addition products of hydrogenated bisphenol F; diols, for example, polyesterdiols such as bis(hydroxyethyl)terephthalate, etc., at least trihydric alcohols such as glycerol, trimethylolpropane, trimethylolethane, diglycerol, triglycerol, 1,2,6-hexanetriol, pentaerythritol, dipentaerythritol, sorbitol, mannitol, etc. Such polyols can be used singly or as a combination of two or more.

The carboxyl-containing resin (a) preferably has a weight average molecular weight of about 500 to about 100,000, and more preferably about 1,000 to about 90,000. The carboxyl content of resin (a) is preferably about 5 to about 700 mgKOH/g, calculated as the acid value of the resin. When the carboxyl content is less than 5 mg KOH/g, the cured coating film may have insufficient degrees of crosslinking. When the carboxyl content is more than 700 mgKOH/g, the coating composition tends to have low storage stability. Thus the resin (a) preferably has an acid value in the range of about 10 to about 600 mgKOH/g.

The resin (a) preferably has a glass transition temperature (Tg) of about 0 to about 200°C. When the Tg is less than 0°C, the coating film becomes sticky, so that foreign matter, dust, etc. tend to easily adhere thereto, thus being difficult to handle. When the Tg is more than 200°C, the cured coating film tends to be fragile. Thus the Tg is preferably in the range of about 5°C to about 200°C.

### Hydroxyphenyl-containing resin (b)

Resin (b) contains hydroxyphenyl group(s) and is capable of forming a coating film. Examples of compounds usable as resin (b) include condensates of a phenol compound and a carbonyl compound; homopolymers of a hydroxyl-containing vinyl aromatic compound; copolymers of a hydroxyl-containing vinyl aromatic compound and other monomer(s) copolymerizable therewith; etc.

The phenol compound/carbonyl compound condensate can be prepared by a known condensation reaction. When a phenol compound and a carbonyl compound are subjected to a condensation reaction using an alkali catalyst, a resol phenolic resin is usually obtained. When the condensation is performed using an acid catalyst, a novolac phenolic resin is usually obtained. The phenolic resin has an increased molecular weight as the condensation proceeds. The condensation is usually allowed to proceed for about 1 to about 24 hours to provide a phenolic resin with a weight average molecular weight of about 500 to about 100,000.

Examples of such phenol compounds include monofunctional or polyfunctional phenol compounds, alkylphenol compounds, etc. Examples of carbonyl compounds include formaldehyde, acetone, etc.

Examples of monofunctional or polyfunctional phenol compounds include compounds containing 1 to 3 hydroxyl groups on the benzene ring, such as phenol, o-cresol, m-cresol, p-cresol, 3,5-xylenol, 2,6-xylenol, 2,4-xylenol, catechol, resorcinol, pyrogallol, bisphenol A, etc. Examples of alkylphenol compounds include alkylphenol compounds whose alkyl moiety contains 1 to 10 carbon atoms, and preferably 1 to 4 carbon atoms, such as p-isopropylphenol, p-tert-butylphenol, p-tert-amylphenol, p-tert-octylphenol, etc.

Such a homopolymer of a hydroxyl-containing vinyl aromatic compound and such a copolymer of a hydroxyl-containing vinyl aromatic compound and other monomer(s) copolymerizable therewith can be prepared by subjecting the respective specified monomers to a known radical polymerization reaction.

Examples of hydroxyl-containing vinyl aromatic compounds include p-hydroxystyrene, m-hydroxystyrene, o-hydroxystyrene, 3-methoxy-4-hydroxystyrene, 3,5-dibromo-4-hydroxystyrene, etc. Examples of other monomers copolymerizable with such hydroxyl-containing vinyl aromatic compounds include C₁₋₁₂ alkyl esters of (meth)acrylic acid such as methyl (meth)acrylate, ethyl (meth)acrylate, propyl (meth)acrylate, butyl (meth)acrylate, hexyl (meth)acrylate, octyl (meth)acrylate, 2-ethylhexyl (meth)acrylate, nonyl (meth)acrylate, decyl (meth)acrylate, etc.; C₂₋₆ hydroxyalkyl esters of (meth) acrylic acid such as hydroxyethyl (meth)acrylate, 2-hydroxypropyl (meth) acrylate, 3-hydroxypropyl (meth)acrylate, hydroxybutyl (meth)acrylate, etc.; vinyl aromatic compounds such as styrene, α-methylstyrene, p-tert-butylstyrene, etc.; vinyl acetate, (meth)acrylonitrile, (meth)acrylamide, vinyl pyrrolidone, etc. Such monomers can be used singly or in a combination of two or more.

The homomonomer of a hydroxyl-containing vinyl aromatic compound and the copolymer of a hydroxyl-containing vinyl aromatic compound and other monomer(s) copolymerizable therewith preferably have a weight average molecular weight of about 500 to about 100,000, and more preferably about 1,000 to about 80,000.

The hydroxyphenyl content of resin (b) is about 5 to about 600 mgKOH/g, calculated as the hydroxyl value of the resin. When the hydroxyphenyl content is less than 5 mgKOH/g, the coating film may have insufficient degrees of crosslinking. When the hydroxylphenyl content is more than 600 mgKOH/g, the crosslinked film tends to be fragile. Thus the resin (b) preferably has a hydroxyl value of about 10 to about 550 mgKOH/g.

The resin (b) preferably has a glass transition temperature (Tg) of about 0°C to about 200°C. When the Tg is less than 0°C, the coating film becomes sticky, so that foreign matter, dust, etc. tend to easily adhere thereto, thus being difficult to handle. When the Tg is more than 200°C, the cured coating film tends to be fragile. Thus, the Tg is preferably in the range of about 5 to about 200°C.

### Carboxyl- and hdroxyphenyl-containing resin (c)

Resin (c) contains carboxyl group(s) and hydroxyphenyl group(s) and is capable of forming a coating film.

Examples of compounds usable as resin (c) include copolymers of a hydroxyl-coritaining vinyl aromatic compound and a carboxyl-containing polymerizable unsaturated monomer; copolymers of a hydroxyl-containing vinyl aromatic compound, a carboxyl-containing polymerizable unsaturated monomer, and other monomer(s) copolymerizable therewith; and polymers obtained by condensation of a phenolcarboxylic acid or a mixture of a phenolcarboxylic acid and a phenol with formaldehyde.

Such a copolymer of a hydroxyl-containing vinyl aromatic compound and a carboxyl-containing polymerizable unsaturated monomer, and such a copolymer of a hydroxyl-containing vinyl aromatic compound, a carboxyl-containing polymerizable unsaturated monomer, and other monomer(s) copolymerizable therewith can be prepared by subjecting the respective specified monomers to a known radical polymerization reaction.

Examples of such hydroxyl-containing vinyl aromatic compounds include p-hydroxystyrene, m-hydroxystyrene, o-hydroxystyrene, 3-methoxy-4-hydroxystyrene, 3,5-dibromo-4-hydroxystyrene, etc. Examples of carboxyl-containing polymerizable unsaturated monomers include acrylic acid, methacrylic acid, crotonic acid, itaconic acid, etc. Examples of other monomers copolymerizable therewith include C₁₋₁₂ alkyl esters of (meth)acrylic acid such as methyl (meth)acrylate, ethyl (meth)acrylate, propyl (meth)acrylate, butyl (meth)acrylate, hexyl (meth)acrylate, octyl (meth)acrylate, 2-ethylhexyl (meth)acrylate, nonyl (meth)acrylate, decyl (meth)acrylate, etc.; C₂₋₆ hydroxyalkyl esters of (meth)acrylic acid such as hydroxyethyl (meth)acrylate, 2-hydroxypropyl (meth)acrylate, 3-hydroxypropyl (meth)acrylate, hydroxybutyl (meth)acrylate, etc.; vinyl aromatic compounds such as styrene, α-methylstyrene, p-tert-butylstyrene, etc.; vinyl acetate, (meth)acrylonitrile, (meth)acrylamide, vinyl pyrrolidone, etc. Such monomers can be used singly or in a combination of two or more.

The polymer obtained by condensation of a phenolcarboxylic acid or a mixture of a phenolcarboxylic acid and a phenol with formaldehyde can be prepared by a known condensation reaction. Examples of phenolcarboxylic acids include hydroxybenzoic acid, gallic acid, resorcinol acid, etc. Examples of phenols include phenol, mono- or di-C₁₋₁₈ alkylphenols, mono- or di-C₁₋₁₈ alkylphenols, resorcinol, catechol, etc.

The resin (c) preferably has a weight average molecular weight of about 500 to about 100,000. The carboxyl content of resin (c) is preferably about 5 to about 700 mg KOH/g, calculated as the acid value of the resin. The hydroxyphenyl content of resin (c) is preferably about 5 to about 600 mg KOH/g, calculated as the hydroxyl value of the resin. When the carboxyl content is less than 5 mg KOH/g, the coating film may have insufficient degrees of crosslinking. When the carboxyl content is more than 700 mgKOH, the coating composition tends to be have reduced storage stability. When the hydroxyphenyl content is less than 5 mg KOH/g, the coating film may have insufficient degrees of crosslinking. When the hydroxyl content is more than 600 mgKOH, the coating composition tends to have reduced storage stability. Thus, the resin (c) preferably has an acid value of about 10 to about 600 mg KOH/g, and a hydroxyl value of about 10 to about 550 mgKOH/g.

The resin (c) preferably has a glass transition temperature (Tg) of about 0°C to about 200°C. When the Tg is less than 0°C, the coating film becomes sticky, so that foreign matter, dust, etc. tend to easily adhere thereto, thus being difficult to handle. When the Tg is more than 200°C, the cured coating film tends to be fragile. Thus, the Tg is preferably in the range of about 5°C to about 200°C.

### Preparation of thermosetting resin composition I

In thermosetting resin composition I, the mixing ratio of the polyepoxy compound (B) of the invention to the carboxyl-and/or hydroxyphenyl-containing resin (A) is preferably such that about 1 to about 3,000 parts by weight, and more preferably about 1.1 to about 2,500 parts by weight, of the compound (B) is mixed per 100 parts by weight of the solids content of the resin (A). When the amount of compound (B) is less than 1 part by weight, the composition may have insufficient curability. When the amount of compound (B) is more than 3,000 parts by weight, the unreacted epoxy groups remain in the cured coating film, so that precipitation and leaching of the unreacted epoxy compound occur, thus being undesirable.

The thermosetting resin composition I of the invention may contain a curing accelerator. Examples of such curing accelerators include various quaternary ammonium salts which vary in their cationic portion. For example, tetraalkyl ammonium salts, trialkyl aralkyl ammonium salts, etc. are easily available for use as such quaternary ammonium salts. Quaternary ammonium salts of nitrogen-containing heterocyclic compounds, such as pyridine, piperidine, piperazine, morpholine, etc. can also be used. Specific examples include tetrabutylammonium, tetramethylammonium, tetraethylammonium, trimethylbenzylammonium, tetrapropylammonium, tetrahexylammonium, tetraoctylammonium, tetradecylammonium, tetrahexadecylammonium, triethylhexylammonium, 2-hydroxyethyltrimethylammonium (choline), methyltrioctylammonium, cetyltrimethylammonium, 2-chloroethyltrimethylammonium, methylpyridinium, etc.

The counter anion of such quaternary ammonium salts can be selected from those capable of forming stable salts with said cationic portion. Examples of counter anions include halides, carboxylates, sulfonates, sulfates, phosphates, etc. Specific examples thereof include acetate, laurate, glycolate, benzoate, salicylate, maleate, phthalate, fluoride, chloride, bromide, iodide, methanesulfonate, p-toluenesulfonate, dodecylbenzenesulfonate, triflate, nitrate, sulfate, methosulfate, phosphate, di-t-butyl phosphate, etc.

Examples of curing accelerators other than quaternary ammonium salts include imidazole compounds such as 2-methylimidazole, 2,4-dimethylimidazole, 2-ethyl-4-methylimidazole, 2-phenylimidazole, 2-phenyl-4-methylimidazole, 2-heptadecylimidazole, etc.; tertiary amine compounds such as triethylamine, benzyldimethylamine, α-methylbenzyldimethylamine, 2-(dimethylaminomethyl)phenol, 2,4,6-tris(dimethylaminomethyl)phenol, 1,8-diazabicyclo(5,4,0)undecene-7, etc.; organometallic compounds such as zirconium tetramethoxide, zirconium tetrapropoxide, tetrakis(acetylacetonato)zirconium, tri(acetylacetonato)aluminum, etc.; organic phosphine compounds such as triphenylphosphine, trimethylphosphine, triethylphosphine, tributylphosphine, tri(p-methylphenyl)phosphine, tri(nonylphenyl)phosphine, etc.

Such curing accelerators can be used singly or in a combination of two or more. The amount of curing accelerators is preferably about 0.1 to about 10 wt.%, based on the total solids content of the carboxyl- and/or hydroxylphenyl-containing resin (A) and polyepoxy compound (B).

The thermosetting resin composition I of the invention may contain pigments such as coloring pigments, luster pigments, extender pigments, etc.; inorganic fillers; etc., if necessary.

Examples of coloring pigments include inorganic pigments such as titanium dioxide, iron oxide, carbon black, etc.; and organic pigments such as phthalocyanine blue, quinacridone red, perylene red, phthalocyanine green, etc. Examples of luster pigments include aluminum flakes, mica flakes, color mica flakes, etc. Examples of extender pigments include barium sulfate, calcium carbonate, talc, clay, etc.

Examples of inorganic fillers include reinforced silica, aluminum oxide, silicon nitride, aluminum nitride, silica-coated aluminum nitride, boron nitride, etc.

The thermosetting resin composition I of the invention can be prepared by mixing the above components according to a known method. When the resin component to be used is in the form of an organic solvent solution, emulsion, etc., such a component can be mixed as is. When using a pigment, the pigment may be used in the form of a paste, which is formed by mixing the pigment with a dispersion resin. If necessary, an organic solvent, water, or a mixture thereof may be added when mixing the components.

Examples of organic solvents that can be used in the thermosetting resin composition I of the invention include aromatic solvents such as toluene, xylene, etc.; ester solvents such as ethyl acetate, propyl acetate, butyl acetate, methoxybutyl acetate, amyl acetate, methylcellosolve acetate, cellosolve acetate, diethylene glycol monomethyl ether acetate, carbitol acetate, etc.; ether solvents such as dioxane, ethylene glycol diethylether, ethylene glycol dibutyl ether, etc.; and ketone solvents such as acetone, methyl ethyl ketone, methyl isobutyl ketone, cyclohexanone, etc. Such organic solvents can be used singly or as a mixture of two or more.

The thermosetting resin composition I of the invention can be an organic solvent-based resin composition or an aqueous resin composition. It is usually preferable that the solids content of the resin composition be about 20 to about 80 wt.%.

### Thermosetting resin composition II

Thermosetting resin composition II comprises the polyepoxy compound (B) as an epoxy resin component and further contains a curing agent (C).

### Curing agent (C)

Curing agent (C) acts to cure the polyepoxy compound (B). Examples of compounds preferably used as curing agent (C) include acid anhydride, polycarboxylic acid, polyphenol compounds, nitrogen-containing compounds, cationic polymerization catalysts, sulfur-containing compounds, etc. Such curing agents can be used singly or as a mixture of two or more. Specific examples of preferable curing agents are described below.

### (1) Acid anhydrides

Examples of usable acid anhydrides include tetrahydrophthalic anhydride, hexahydrophthalic anhydride, methyltetrahydrophthalic anhydride, methylhexahydrophthalic anhydride, methylnadic acid anhydride, hydrogenated methylnadic acid anhydride, trialkyltetrahydrophthalic anhydride, phthalic anhydride, methylcyclohexane tetracarboxylic dianhydride, trimellitic anhydride, pyromellitic anhydride, benzophenonetetracarboxylic anhydride, dodecenylsuccinic anhydride, ethylene glycol trimellitic anhydride, chlorendic anhydride, etc.

### (2) Polycarboxylic acid compounds

Examples of usable polycarboxylic acid compounds include aromatic polybasic acids such as phthalic acid, isophthalic acid, terephthalic acid, 4,4'-biphenyldicarboxylic acid, 2,6-naphthalenedicarboxylic acid, tetrachlorophthalic acid, 4,4'-diphenylmethanedicarboxylic acid, trimellitic acid, trimesic acid, pyromellitic acid, etc.; saturated or unsaturated aliphatic polybasic acids such as succinic acid, glutaric acid, adipic acid, azelaic acid, sebacic acid, dodecanedicarboxylic acid, suberic acid, pimeric acid, maleic acid, fumaric acid, itaconic acid, brassylic acid, citraconic acid, etc.; and alicyclic polybasic acids such as hexahydrophthalic acid, hexahydroisophthalic acid, hexahydroterephthalic acid, hexahydrotrimellitic acid, methylhexahydrophthalic acid, methylhexahydroterephthalic acid, tetrahydrophthalic acid, tetrahydroisophthalic acid, methyltetrahydrophthalic acid, endomethylene tetrahydrophthalic acid, methylendomethylene tetrahydrophthalic acid, hexachloroendomethylene tetrahydrophthalic acid, etc. Other examples of compounds usable as polybasic acid components include ester-forming reactive derivatives of such polybasic acids, for example, C₁₋₆ lower alkyl esters such as dimethyl esters of polybasic acids.

The carboxyl- and hydroxyphenyl-containing resin and carboxyl-containing resin of the composition I are excluded from the examples of polycarboxylic acids usable as curing agents in the thermosetting resin composition II; however, any carboxyl-containing resins other than the above resins can be used as the curing agent.

### (3) Polyphenol compounds

Examples of polyphenol compounds include products of condensation of a carbonyl compound with a monosubstituted phenol derivative such as resorcinol, bisphenols, phenol, cresol, etc. Examples of bisphenols include bisphenol A, bisphenol B, bisphenol F, etc.

The carboxyl- and hydroxyphenyl-containing resin and hydroxyphenyl-containing resin of the composition I are excluded from the examples of polyphenol compounds usable as curing agents in the thermosetting resin composition II; however, any polyphenol resins other than the above resins can be used as the curing agent.

### (4) Nitrogen-containing compound

Examples of nitrogen-containing compounds include amine compounds, amide compounds, imidazole compounds, etc.

### (i) Amine compound

Examples of amine compounds include aromatic amine compounds, aliphatic amine compounds, alicyclic amine compounds, modified amine compounds, etc.

Examples of aromatic amine compounds include m-xylenediamine, diaminodiphenylmethane, diaminodiphenylsulfone, m-phenylenediamine, fluorenediamine, m-phenylenediamine, p,p'-aminodiphenylmethane, diaminodiphenylsulfone, benzyldimethylamine, etc.

Examples of aliphatic amine compounds include ethylenediamine, diethylenetriamine, triethylenetetramine, tetraethylenepentamine, diethylaminopropylamine, pentanediamine, aminoethylpiperazine, etc.

Examples of alicyclic amine compounds include isophoronediamine, N-dimethylcyclohexylamine, 1,3-bis(aminomethyl)cyclohexane, 4,4'-diaminodicyclohexylmethane, etc.

Examples of modified amine compounds include addition products of a monoepoxy compound such as butyl glycidyl ether, phenyl glycidyl ether, etc., with an aliphatic polyamine such as ethylenediamine, etc.; addition products of an aliphatic polyamine such as ethylenediamine, etc. with ethylene oxide; addition products formed by cyanoethylation of an aliphatic polyamine such as ethylenediamine with acrylonitrile; amine addition products obtained by reaction of a diepoxy compound with a large excess of a polyamine to add 2 polyamine molecules per molecule of the epoxy compound; reaction products of a fatty acid dimer with diamine; ketimine, polyamide resins, BF₃-amine complex, etc.

### (ii) Amide compounds

Examples of amide compounds include dicyandiamide, adipic acid dihydrazide, sebacic acid dihydrazide, isophthalic acid dihydrazide, etc.

### (iii) Imidazole compounds

Examples of imidazole compounds include imidazole, 2-ethylimidazole, 2-ethyl-4-methylimidazole, etc.

### (5) Cationic polymerization catalysts

Examples of compounds usable as catalyst (C) include cationic polymerization catalysts. Examples of such catalysts include Lewis acid salts of aromatic diazonium, aromatic sulfonium, aromatic iodonium, and metallocene compounds. Examples of Lewis acid components include BF₄, PF₆, AsF₆, SbF₆, B(C₆H₅)₄, etc.

### (6) Sulfur-containing compounds

Examples of sulfur-containing compounds usable as curing agent (C) include thiokol (polysulfide), polymercaptan, etc.

Examples of compounds preferably used as curing agent (C) of the invention include acid anhydrides such as hexahydrophthalic anhydride, methylhexahydrophthalic anhydride, methylnadic anhydride, maleic anhydride, etc.; polycarboxylic acids such as phthalic acid, isophthalic acid, terephthalic acid, 4,4'-biphenyl dicarboxylic acid, etc.; polyphenol compounds such as resorcinol, bisphenols, etc.; nitrogen-containing compounds such as amine compounds, amide compounds, imidazole compounds, etc.; and cationic polymierization catalysts such as Lewis acid salts of aromatic diazonium, Lewis acid salts of aromatic sulfonium, Lewis acid salts of aromatic iodonium, Lewis acid salts of metallocene compounds, etc. Further, a combination of two or more compounds selected from such acid anhydrides, polycarboxylic acids and nitrogen-containing compounds are preferably used.

### Preparation of thermosetting resin composition II

In thermosetting resin composition II of the invention, the amount of curing agent (C) is preferably about 0.1 to about 500 parts by weight, and more preferably about 0.5 to 400 parts by weight, per 100 parts by weight of polyepoxy compound (B). When the amount of curing agent (C) is less than 0.1 parts by weight, unreacted epoxy groups remain in the cured product, so that precipitation and leaching of the unreacted epoxy compound may occur. When the amount of curing agent (C) is more than 500 parts by weight, the composition may have insufficient curability, thus being undesirable.

The thermosetting resin composition II of the invention may contain a curing accelerator, if necessary. Examples of curing accelerators include imidazole compounds, tertiary amine compounds, organometallic compounds, organic phosphine compounds, etc. Specific examples of such compounds include those mentioned as examples of curing accelerators in the thermosetting resin composition I.

When the thermosetting resin composition II contains acid anhydride or polycarboxylic acid as the curing agent (C), a quaternary ammonium salt can be added as a curing accelerator. Examples of quaternary ammonium salts and counter anions thereof include those mentioned as examples of curing accelerators in the thermosetting resin composition I.

When using such a curing accelerator, the amount of curing accelerator is about 0.01 to about 15 parts by weight, and more preferably about 0.1 to about 10 parts by weight, per 100 parts by weight of polyepoxy compound (B).

If necessary, the thermosetting resin composition II of the invention may contain pigments such as coloring pigments, luster pigments, extender pigments, etc; inorganic fillers; etc. Specific examples of such pigments and inorganic fillers include those mentioned as examples of components in the thermosetting resin composition I.

The thermosetting resin composition II of the invention can be prepared by mixing the above-mentioned components according to a known method. When the resin component to be used is in the form of an organic solvent solution, emulsion, etc., such a component can be mixed as is. When using a pigment, the pigment may be used in the form of a paste, which is formed by mixing the pigment with a dispersion resin. If necessary, an organic solvent, water, or a mixture thereof may be added when mixing the components.

Specific examples of organic solvents usable for the thermosetting resin composition II of the invention include those mentioned in the preparation of the thermosetting resin composition I.

The thermosetting resin composition II of the invention can be an organic solvent-based resin composition or an aqueous resin composition. It is usually preferable that the solids content of the resin composition be about 20 to about 80 wt.%.

### Use of the thermosetting resin compositions, cured product thereof and method of removing the cured product

Thermosetting resin compositions I and II can be used as starting materials for coatings, adhesives, printing inks, molded products, etc.

Since the thermosetting resin compositions I and II (hereinafter sometimes simply referred to as "thermosetting resin composition") can be removed by post-heating cured products thereof at a temperature higher than the curing temperature, the compositions are advantageously used as materials for coating, adhesion or printing by application to substrates that need to be recycled or reused. Known methods can be used to form such coating film, adhesive film or printing film using the thermosetting resin composition.

Examples of coating films formed by using the resin composition as a coating composition include undercoat films, intermediate coating films, and topcoat films of automobile parts, etc. Adhesive films formed by using the resin composition as an adhesive include, for example, adhesive layers formed by overlaying and adhering adherends of, for example, automobile accessories, office equipment, household electrical goods, building materials, electronics products, etc.

The substrate to be coated by or adhered to using the thermosetting resin composition of the invention as a coating composition, adhesive, etc. is not particularly limited, and examples include resin molded products, fibers, metal materials, inorganic materials, etc.

Examples of resin molded products include automobile exterior parts such as automobile bumpers, wheel caps, body side mouldings, etc.; automobile interior parts such as instrument panels, levers, linings, etc.; electric products such as cleaners, washers, refrigerators, luminaire, audio equipment, air-conditioners, microwave ovens, cleaners, televisions, personal computers, etc.; various daily-use products such as fixed shelf units, housing cases, etc.

Examples of metal material products include automobile exterior parts; electronic equipment such as personal computers, etc.; office equipment such as printers, copying machines, phones, etc.; housing articles such as furniture, building materials, sealing materials, etc.; electronics products such as liquid crystal panels, plasma displays, semiconductors, printed circuit boards, integrated circuits, etc.; battery packages such as rechargeable batteries, etc.; canned products; vehicle bodies; etc.

Examples of such substrate materials to be coated or adhered include olefin resins such as polyethylene, polypropylene, etc.; thermoplastic polyester resins such as polyethylene terephthalate, polybutylene terephthalate, etc.; engineering plastics such as a polyamide, polyimide, polycarbonate, polyphenylene ether, polyoxymethylene, etc.; styrene resins such as acrylonitrile-styrene copolymers, acrylonitrile-styrenebutadiene copolymers, etc.; polyether resins; epoxy resins; polyurethane resins; ABS resins; rubber; synthetic fibers such as nylon, polyester fibers, acrylic fibers, polyurethane fibers, vinylon, etc.; natural fibers such as cotton, hemp, silk, etc.; semi-synthetic fibers such as viscose rayon, cellulose acetate, etc.; natural materials such as wood, paper, leather, etc.; metals such as iron, steel, gold, silver, copper, aluminum, titanium, zinc, magnesium, tin, etc.; inorganic materials such as glass, gypsum, pottery, ceramics, etc. Among these materials, resins and metals are preferable.

When the thermosetting coating composition of the invention is used as a coating, adhesive, ink, or like composition, the method of application to a substrate is not particularly limited. Examples of usable methods include known coating methods and printing methods such as air spray coating, airless spray coating, rotary atomization coating, electrostatic coating, curtain coating, bar coating, spin coating, roll coating, curtain flow coating, gravure printing, relief printing, screen printing, brush coating, dip coating, electrodeposition coating, etc. The coating composition of the invention used as a coating, adhesive, ink, or like composition is preferably applied to a film thickness of about 5 to about 100 µm, when cured.

The thermosetting resin composition of the invention is preferably heat cured at about 25 to about 180°C for about 5 to 180 minutes. A cured product of the composition of the invention can be thereby formed as a cured coating film, cured adhesive film, or cured printing layer, on the substrate.

The method of removing a cured product of the invention comprises heating the whole or part of a cured product, which has been formed by heat curing the thermosetting rein composition, at a temperature higher than the heat-curing temperature to thermally cleave a tertiary ester bond derived from the polyepoxy compound (B) and then removing the heated portion of the cured product by using or not using a treatment agent. Organic solvents, etc. can be used as such a treatment agent.

By removing a cured product from a coated or adhered substrate, etc. according to the above-mentioned removal method, the substrate, etc. can be easily recycled.

The method of removing a cured coating film formed on a substrate by using the thermosetting resin composition of the invention as a coating composition is described below.

To remove a cured coating film at the time of recycling the substrate, post-heating can be performed at a temperature higher than the heat-curing temperature of the cured coating film, for example, about 180°C to about 300°C for about 0.5 to about 60 minutes so as to thermally cleave the polyepoxy-compound-(B)-derived tertiary ester bond in the cured coating film and destroy the crosslinking structure, followed by dipping in an organic solvent used as a treatment agent, thereby easily removing the cured coating film. Cured coating films, cured adhesive films, etc. formed on scrap can be easily removed by this method.

Examples of organic solvents used as treatment agents include commonly used organic solvents. Specific examples include aliphatic hydrocarbons such as hexane, heptane, petroleum benzine, cyclohexane, etc.; aromatic hydrocarbons such as benzene, xylene, ethylbenzene, etc.; halogen hydrocarbons such as methylene chloride, carbon tetrachloride, trichloroethane, chloroform, etc.; alcohols such as methanol, ethanol, n-propanol, etc.; ethers such as ethyl ether, tetrahydrofuran, etc.; ketones such as an acetone, methyl ethyl ketone, cyclohexanone, etc.; esters such as methyl formate, n-propyl acetate, etc.; polyhydric alcohol derivatives such as ethylene glycol monoethyl ether, etc.; fatty acids, such as acetic acid, etc.; phenol solvents such as phenol, cresol, xylenol, etc.; nitrogen-containing solvents such as N,N-dimethylacetamide, N,N-diethylacetamide, N,N-dimethylformamide, N,N-diethylformamide, N-methyl-2-pyrrolidone, 1,3-dimethyl-2-imidazolidinone, N-methylcaprolactam, etc.; sulfur-containing solvents such as dimethylsulfoxide, diethylsulfoxide, dimethylsulfone, diethylsulfone, etc. Such organic solvents can be used singly or in a combination of two or more.

### EFFECT OF THE INVENTION

The present invention achieves the following remarkable effects:
(1) Since the polyepoxy compound of the invention has highly reactive olefin oxide groups such as methylglycidyl, glycidyl, etc., thermosetting resin compositions containing this compound have excellent curability. In particular, thermosetting resin compositions containing the polyepoxy compound of formula (II) have excellent curability because the compound contains 2 to 8 olefin oxide groups such as methylglycidyl, glycidyl, etc. per molecule.
(2) Since the polyepoxy compound of the invention contains a tertiary ester group of formula (I) which has excellent thermal cleavability, cured coating films, i.e., cured products of thermosetting resin compositions containing this polyepoxy compound have excellent thermal cleavability and can be easily removed by post-heating. In particular, cured coating films of thermosetting resin compositions containing the polyepoxy compound of formula (II) have excellent thermal cleavability because the compound contains 2 to 8 tertiary ester groups per molecule.
(3) Therefore, due to the thermosetting resin composition of the invention, cured coating films, etc. formed by applying the composition to a substrate to be coated or adhered and heating can be decomposed by post-heating at a temperature higher than the curing temperature and easily removed by, for example, using an organic solvent, etc., at the time of recycling the substrate. Since this composition enables easy removal of cured coating films, etc. from coated resin scrap, metal scrap, etc., by a simple process without the need to use any special equipment, it can greatly contribute to recycling of vehicles, electronic equipment, can products, etc.

### BRIEF DESCRIPTION OF THE DRAWINGS

[Fig. 1] Fig. 1 shows a NMR spectrum of the polyepoxy compound obtained in Example 1.
[Fig. 2] Fig. 2 shows an IR spectrum of the polyepoxy compound obtained in Example 1.

### BEST MODE FOR CARRYING OUT THE INVENTION

Production Examples, Examples, and Comparative Examples are given below to illustrate the invention in more detail; however, the scope of the invention is not limited to these examples. All "parts" and "%" used herein are by weight unless otherwise specified.

### Production Example 1

### Production of 4-allyloxy-2-methyl-butan-2-ol

After a 5 liter glass container was purged with nitrogen, 67.2 g of sodium hydride was placed therein and 2,000 ml of dehydrated tetrahydrofuran was added. Then 192.5 g of 3-methyl-1,3-butanediol was gradually added dropwise while observing the generation of hydrogen. After completion of the dropwise addition, the reaction mixture was stirred at room temperature overnight. Subsequently, 235.7 g of allyl chloride was added and the mixture was then stirred for 3 days. After completion of the reaction, tetrahydrofuran used as the reaction solvent was removed using a rotatory evaporator. Then 800 ml of water was added to the reaction solution to inactivate the residual sodium hydride, and the reaction product was extracted with 1,200 ml of hexane. This extraction with hexane was performed a total of three times.

Subsequently, the organic layer was dried over anhydrous magnesium sulfate and concentrated. The concentrated product was distilled under reduced pressure (1 torr, 60°C) to give 200 g of 4-allyloxy-2-methyl-butan-2-ol.

### Production Example 2

### Production of carboxyl-containing resin (A-1)

A mixture of 7.8 parts of acrylic acid, 50 parts of methyl methacrylate, 12.2 parts of ethyl acrylate, 30 parts of phenoxyethylene glycol acrylate (trade name "Aronix M-101", product of Toagosei Co., Ltd.), and 1 part of tertiary butylperoxy 2-ethylhexanoate (trade name "PERBUTYL 0", product of NOF corporation), as a polymerization initiator, was copolymierized according to a conventional method to give a carboxyl-containing acrylic resin (A-1). The resin had a weight average molecular weight of 60,000, an acid value of 60 mgKOH/g, a glass transition point of 44°C, and a solids content of 60%.

### Production Example 3

### Production of carboxyl- and hydroxyphenyl-containing Resin (A-2)

Six hundred parts of o-hydroxybenzoic acid, 900 parts of o-cresol, 1,145 parts of 30% formalin, 130 parts of deionized water, and 6.5 parts of oxalic acid were placed into a flask and refluxed for 60 minutes. After 13.5 parts of 15% hydrochloric acid was added and refluxed for 40 minutes, 400 parts of about 15°C deionized water was added and the temperature of the flask contents was maintained at about 50°C to precipitate a resin. Further, 400 parts of deionozed water was added and the resin was washed at 50°C, followed by removal of the water layer. After the washing process was repeated three times, the resin was dried at about 120°C under reduced pressure to give a carboxyl- and hydroxyphenyl-containing resin (A-2). The resin had a weight average molecular weight of about 650, an acid value of 157 mgKOH/g, an hydroxyl value of 303 mgKOH/g, and a glass transition point of 130°C.

### Example 1

### Production of polyepoxy compound and curability evaluation

### (1) Production of olefin bond- and tertiary ester-containing compound

Forty grams of terephthalic acid chloride and 400 ml of methylene chloride (solvent) were placed into a 2-liter flask equipped with a stirrer, reflux condenser, thermometer, and one dropping funnel. A solution of 200 g of 4-allyloxy-2-methyl-butan-2-ol, 40 ml of pyridine, 4.8 g of dimethylaminopyridine, and 100 ml of methylene chlorides was placed into the dropping funnel.

The solution was added dropwise to the flask from the funnel at room temperature over a period of 1 hour. After completion of the dropwise addition, the mixture was stirred at room temperature for 1 week. After stirring, the precipitate was removed by filtration, and the residual pyridine and dimethylaminopyridine were neutralized with 500 ml of cooled 2N hydrochloric acid and removed by liquid-phase separation. After washing twice with 10 wt.% aqueous sodium hydrogencarbonate solution and drying over anhydrous magnesium sulfate, the solvent was distilled off. The crude product obtained by distilling off the solvent was purified by distillation at 2 Pa using a high-vacuum thin-film distillation apparatus (a compact high vacuum pumping system "VPC-050" and an air-cooled oil diffusion pump "DPF-050", products of ULVAC KIKO, Inc.) and 2-ethyl-1-hexanol as a heated solvent, thus giving 57.7 g of the desired bis(3-allyloxy-1,1-dimethylpropyl)terephthalate.

### (2) Production of tertiary ester-containing glycidyl ether compound

Twenty grams of bis(3-allyloxy-1,1-dimethylpropyl)terephthalate and 50 ml of methylene chloride (solvent) were placed into a 1-liter flask equipped with a stirrer, reflux condenser, thermometer, and one dropping funnel and cooled to 5°C or lower. A solution of 39 g of 3-chloroperbenzoic acid in 300 ml of methylene chloride was placed into the dropping funnel and added to the flask dropwise over a period of 2 hours. The mixture was stirred at room temperature for 12 hours to complete the reaction. After the reaction, the reaction mixture was cooled to 10°C using an ice bath, and an aqueous sodium thiosulfate solution (1N) was added. The mixture was returned to room temperature and then stirred for a further 1 hour.

Then, 200 ml of methylene chloride was added and the mixture was subjected to liquid-phase separation. The organic layer (methylene chloride layer) was washed with an aqueous sodium hydroxide solution (0.5N) and sequentially washed with an aqueous sodium chloride solution until the organic layer became neutral. The washed organic layer was dried over magnesium sulfate and methylene chloride was removed by distillation to give 23 g of bis(3-glycidylpropyloxy-1,1-dimethylpropyl)terephthalate. This polyepoxy compound was obtained with a yield of 98% and a purity of 98% or higher, as measured by liquid chromatography. This polyepoxy compound was a colorless liquid with an epoxy equivalent of 230.

### (3) The polyepoxy compound obtained above in (2) was analyzed using its NMR spectrum (nuclear magnetic resonance spectrum) and IR spectrum (infrared absorption spectrum). The analytical methods used were as follows.

### 1. NMR spectrum

The NMR spectrum of the reaction product was obtained as ¹H NMR spectral data using a "EX400FT-IR" (400 MHz) spectrometer manufactured by JEOL Ltd., using tetramethylsilane as a reference material and dimethylsulfoxide-d₆ as a solvent.

### 2. IR spectrum

The IR spectrum was obtained by applying the reaction product to a potassium bromide plate and measured using a Fourier transform infrared spectrometer "FT/IR-610" manufactured by JASCO Corporation.

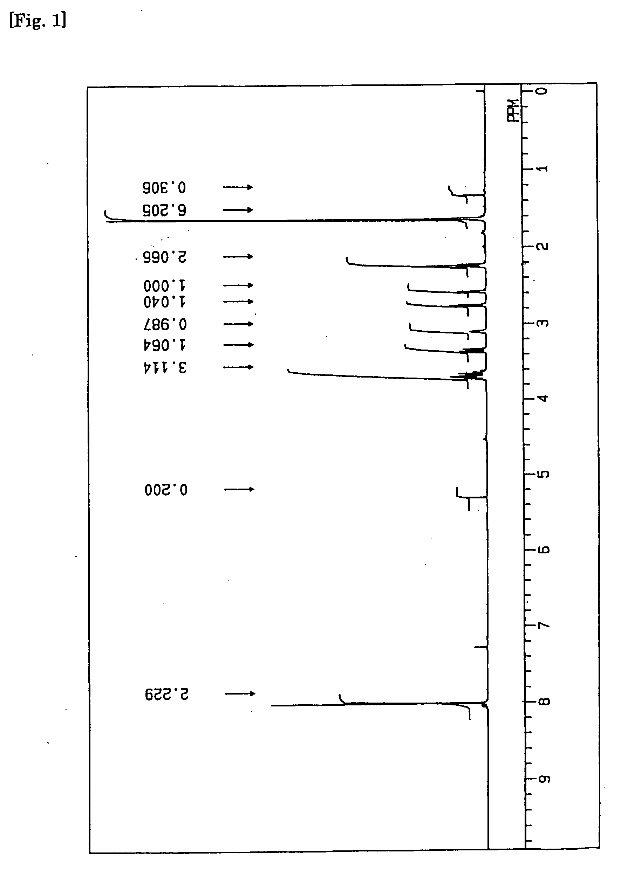

Fig. 1 shows the ¹H NMR spectral results of the reaction product. In Fig. 1, the signals of the ¹H NMR spectrum are as follows:
δ (ppm): 8.02 (s 2H),
δ (ppm): 3.63 to 3.74 (m 3H),
δ (ppm): 3.33 to 3.78 (m 1H),
δ (ppm) : 3.11 (m 1H),
δ (ppm): 2.76 (m 1H),
δ (ppm): 2.57 to 2.59 (m 1H),
δ (ppm): 2.25 (t 2H J=6.8),
δ (ppm) : 1.64 (s 6H).

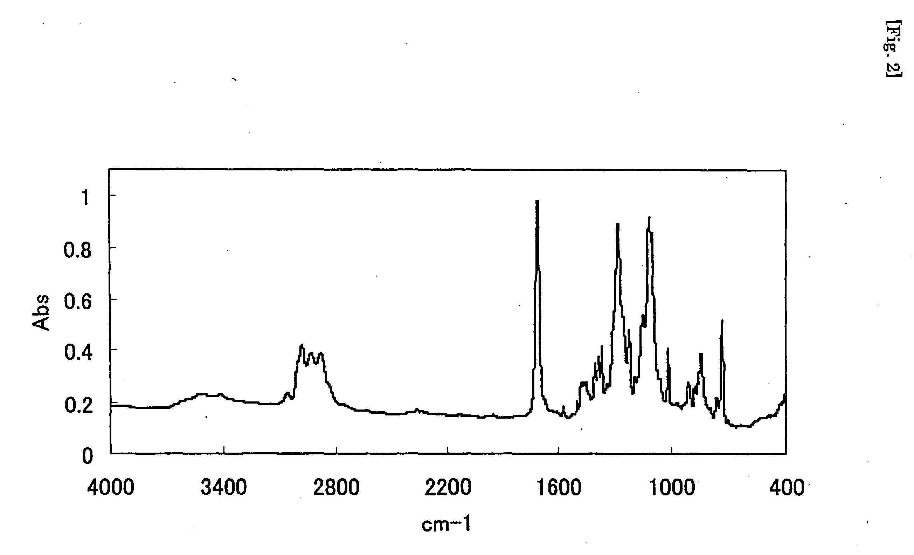

Fig. 2 shows the IR spectral results of the reaction product. In Fig. 2, the peak absorption wavelengths are as follows:
2977 cm⁻¹: absorption by C-H,
2929 cm⁻¹: absorption by C-H,
1714 cm⁻¹: absorption by C=O,
1283 cm⁻¹: absorption by C-O-C,
1119 cm⁻¹: absorption by C-O-C,
1017 cm⁻¹: absorption by C-O-C,
911 cm⁻¹: absorption by epoxy C-O-C,
843 cm⁻¹: absorption by epoxy C-O-C.

The above analysis results confirm that the reaction product obtained by the above reaction is the polyepoxy compound of the invention represented by formula 2.

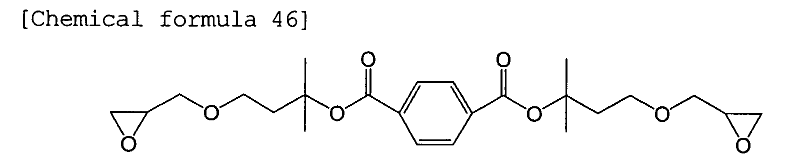

### (4) Curability evaluation

A 9.4 g quantity of methylhexahydrophthalic anhydride and 0.1 g of 2-ethyl-4-imidazole were mixed with 14 g of the obtained bis(3-glycidylpropyloxy-1,1-dimethylpropyl)terephthalate to give a thermosetting resin composition of the invention. This composition was injected into a 100 µm gap between a tin plate and a tetrafloroethylene plate and heated in a hot-air drying furnace at 160°C for 30 minutes to give a cured coating film.

The curability was evaluated by solvent extraction of the cured coating film separated from the space between the tin plate and the tetrafluoroethylene plate under acetone reflux for 2 hours, followed by evaluation based on the weight change of the coating film remained after the solvent extraction (residual coating film %). The residual coating film % was 96.5% of the original.

A cured coating film prepared in the same manner as above and further post-heated in a hot-air drying furnace at 220°C for 10 minutes was subjected to solvent extraction in the same manner as above. The residual coating film % was 6%; this result confirms that the coating film decomposes at a comparatively low temperature of about 220°C.

### Comparative Example 1

A comparative thermosetting composition was prepared by mixing 17.8 g of methylhexahydrophthalic anhydride and 0.2 g of 2-ethyl-4-imidazole with 20 g of bisphenol A diglycidyl ether. This composition was injected into a 100 µm gap between a tin plate and a tetrafloroethylene plate and heated in a hot-air drying furnace at 160°C for 30 minutes to give a cured coating film.

The curability was evaluated in the same manner as above by solvent extraction of the cured coating film separated from the space between the tin plate and the tetrafluoroethylene plate under acetone reflux for 2 hours, followed by evaluation based on the weight change of the coating film remaining after the solvent extraction (residual coating film %). As a result, the residual coating film % was 98.6%.

A cured coating film prepared in the same manner as above and further post-heated in a hot-air drying furnace at 220°C for 10 minutes was subjected to solvent extraction in the same manner as above. The residual coating film % was 98.5%; this result confirmed that the coating film does not decompose by heating to about 220°C.

### Preparation of thermosetting resin composition I

### Example 2

One hundred parts of a carboxyl-containing acrylic resin (A-1), 24 parts of the polyepoxy compound of formula 2 obtained in Example 1, and 0.7 parts of tetrabutylammonium bromide were dissolved in 223 parts of cyclohexanone to give a thermosetting resin composition of the invention.

This composition was applied to a tin plate using a bar coater to a film thickness of 10 µm (when cured) and heated at 140°C for 30 minutes to cure the coating film. The obtained cured coating film was subjected to a rubbing test using an absorbent cotton immersed in acetone.

Subsequently, this cured film-coated tin plate was post-heated at 220°C for 10 minutes and subjected to the rubbing test.

### Example 3

One hundred parts of a hydroxylphenyl-containing resin (weight average molecular weight: 1,200, hydroxyl value: 519 mgKOH/g), 187 parts of polyepoxy compound of Formula 2 obtained in Example 1, and 2 parts of 2-ethyl-4-imidazole were dissolved in 223 parts of cyclohexanone to give a thermosetting resin composition of the invention.

This resin composition was applied to a tin plate using a bar coater to a film thickness of 10 µm (when cured) and heated at 140°C for 30 minutes to cure the coating film. The obtained cured coating film was subjected to the rubbing test.

Subsequently, this cured film-coated tin plate was post-heated at 220°C for 10 minutes and subjected to the rubbing test.

### Example 4

One hundred parts of carboxyl- and hydroxylphenyl-containing resin (A-2), 192 parts of the polyepoxy compound of Formula 2 obtained in Example 1, and 0.7 parts of tetrabutylammonium bromide were dissolved in 223 parts of cyclohexanone to give a thermosetting resin composition of the invention.

This resin composition was applied to a tin plate using a bar coater to a film thickness of 10 µm (when cured) and heated at 140°C for 30 minutes to cure the coating film. The obtained cured coating film was subjected to the rubbing test.

Subsequently, this cured film-coated tin plate was post-heated at 220°C for 10 minutes and subjected to the rubbing test.

### Comparative Example 2

One hundred parts of a carboxyl-containing acrylic resin (A-1), 18 parts of bisphenol A diglycidyl ether and 0.7 parts of tetrabutylammonium bromide were dissolved in 223 parts of cyclohexanone to give a comparative thermosetting resin composition.

This resin composition was applied to a tin plate using a bar coater to a film thickness of 10 µm (when cured) and heated at 140°C for 30 minutes to cure the coating film. The obtained cured coating film was subjected to the rubbing test.

Subsequently, this cured film-coated tin plate was post-heated at 220°C for 10 minutes and subjected to the rubbing test.

### Comparative Example 3

One hundred parts of a hydroxylphenyl-containing resin (weight average molecular weight: 1,200, hydroxyl value: 519 mgKOH/g), 142 parts of bisphenol A diglycidyl ether, and 2 parts of 2-ethyl-4-imidazole were dissolved in 223 parts of cyclohexanone to give a comparative thermosetting resin composition.

This resin composition was applied to a tin plate using a bar coater to a film thickness of 10 µm (when cured) and heated at 140°C for 30 minutes to cure the coating film. The obtained cured coating film was subjected to the rubbing test.

Subsequently, this film-coated tin plate was post-heated at 220°C for 10 minutes and subjected to the rubbing test.

### Comparative Example 4

One hundred parts of a carboxyl- and hydroxylphenyl-containing resin (A-2), 102 parts of bisphenol A diglycidyl ether, and 0.7 parts of tetrabutylammonium bromide were dissolved in 223 parts of cyclohexanone to give a comparative thermosetting resin composition.

This resin composition was applied to a tin plate using a bar coater to a film thickness of 10 µm (when cured) and heated at 140°C for 30 minutes to cure the coating film. The obtained cured coating film was subjected to a rubbing test.

Subsequently, this cured film-coated tin plate was post-heated at 220°C for 10 minutes and subjected to the rubbing test.

In Examples 2 to 4 and Comparative Examples 2 to 4, the rubbing test was performed according to the following method:
Rubbing test: The cured coating film surface was rubbed with an acetone-dipped absorbent cotton in the forward and reverse directions reciprocally 30 times (i.e., 30 cycles) at maximum. Evaluation was made based on the maximum number of rubbing cycle(s) with which no scratches occurred on the coated surface.

Table 1 shows the results of the rubbing test.
[Table 1]

**Table 1**

| | | Example | | | Comp. Ex. | | |
|---|---|---|---|---|---|---|---|
| | | 2 | 3 | 4 | 2 | 3 | 4 |
| Number of rubbing cycle(s) | After heating at 140°C for 30 min. | 30 | 30 | 30 | 30 | 30 | 30 |
| | After post-heating at 220°C for 10 min. | 1 | 1 | 1 | 30 | 30 | 30 |

### Preparation of thermosetting resin composition II

### Example 5

One hundred parts of the polyepoxy compound of Formula 2 obtained in Example 1, 67 parts of methylhexahydrophthalic anhydride, and 1 part of 2-ethyl-4-imidazole were mixed to give a thermosetting resin composition of the invention.

This resin composition was injected into a 100 µm gap between a tin plate and a tetrafloroethylene plate and heated at 160°C for 30 minutes in a hot-air drying furnace to give a cured coating film. The curability of the composition was evaluated by solvent extraction of the cured coating film separated from the space between the tin plate and the tetrafrluoroethylene plate under acetone reflux for 2 hours, followed by evaluation based on the weight change of the coating film remaining after the solvent extraction (residual coating film %).

The thermal degradability of the cured coating film was evaluated by subjecting a cured coating film prepared in the same manner as above to post-heating at 220°C for 10 minutes in a hot-air drying furnace, followed by solvent extraction and evaluation based on the weight change of the coating film remaining after the solvent extraction (residual coating film %) in the same manner as above.

### Example 6

One hundred parts of the polyepoxy compound of formula 2 obtained in Example 1 and 30 parts of m-xylenediamine were mixed to give a thermosetting resin composition of the invention.

This resin composition was injected into a 100 µm gap between a tin plate and a tetrafloroethylene plate and heated at 160°C for 30 minutes in a hot-air drying furnace to give a cured coating film.

The curability of the composition and thermal degradability of the cured coating film were evaluated in the same manner as in Example 5.

### Example 7

One hundred parts of the polyepoxy compound of formula 2 obtained in Example 1, 37 parts of terephthalic acid, and 1 part of tetrabutylammonium bromide were mixed to give a thermosetting resin composition of the invention.

This resin composition was injected into a 100 µm gap between a tin plate and a tetrafloroethylene plate and heated at 160°C for 30 minutes in a hot-air drying furnace to give a cured coating film.

The curability of the composition and thermal degradability of the cured coating film were evaluated in the same manner as in Example 5.

### Example 8

One hundred parts of the polyepoxy compound of Formula 2 obtained in Example 1, 51 parts of bisphenol A, 1 part of 2-ethyl-4-imidazole were mixed to give a thermosetting resin composition of the invention.

This resin composition was injected into a 100 µm gap between a tin plate and a tetrafloroethylene plate and heated at 160°C for 30 minutes in a hot-air drying furnace to give a cured coating film.

The curability of the composition and thermal degradability of the cured coating film were evaluated in the same manner as in Example 5.

### Comparative Example 5

One hundred parts of bisphenol A diglycidyl ether, 89 parts of methylhexahydrophthalic anhydride, and 1 part of 2-ethyl-4-imidazole were mixed to give a comparative thermosetting resin composition.

This resin composition was injected into a 100 µm gap between a tin plate and a tetrafloroethylene plate and heated at 160°C for 30 minutes in a hot-air drying furnace to give a cured coating film.

The curability of the composition and thermal degradability of the cured coating film were evaluated in the same manner as in Example 5.

### Comparative Example 6

One hundred parts of bisphenol A diglycidyl ether and 40 parts of m-xylenediamine were mixed to give a comparative thermosetting resin composition.

This resin composition was injected into a 100 µm gap between a tin plate and a tetrafloroethylene plate and heated at 120°C for 30 minutes in a hot-air drying furnace to give a cured coating film.

The curability of the composition and thermal degradability of the cured coating film were evaluated in the same manner as in Example 5.

### Comparative Example 7

One hundred parts of bisphenol A diglycidyl ether, 49 parts of terephthalic acid, and 2 parts of tetrabutylammonium bromide were mixed to give a comparative thermosetting resin composition.

This resin composition was injected into a 100 µm gap between a tin plate and a tetrafloroethylene plate and heated at 160°C for 30 minutes in a hot-air drying furnace to give a cured coating film.

The curability of the composition and thermal degradability of the cured coating film were evaluated in the same manner as in Example 5.

### Comparative Example 8

One hundred parts of bisphenol A diglycidyl ether, 67 parts of bisphenol A, and 1 part of 2-ethyl-4-imidazole were mixed to give a comparative thermosetting resin composition.

This resin composition was injected into a 100 µm gap between a tin plate and a tetrafloroethylene plate and heated at 160°C for 30 minutes in a hot-air drying furnace to give a cured coating film.

The curability of the composition and thermal degradability of the cured coating film were evaluated in the same manner as in Example 5.

Table 2 shows the formulations of the resin compositions obtained in Examples 5 to 8 and Comparative Examples 5 to 8, and evaluation results of the curability of the compositions and the thermal degradability of the cured coating films.
[Table 2]

**Table 2**

| | Example | | | | Comp. Ex. | | | |
|---|---|---|---|---|---|---|---|---|
| | 5 | 6 | 7 | 8 | 5 | 6 | 7 | 8 |
| Polyepoxy compound of Formula 2 | 100 | 100 | 100 | 100 | | | | |
| Bisphenol A diglycidyl ether | | | | | 100 | 100 | 100 | 100 |
| Methylhexahydrophthalic anhydride | 67 | | | | 89 | | | |
| m-xylenediamine | | 30 | | | | 40 | | |
| Terephthalic acid | | | 37 | | | | 49 | |
| Bisphenol A | | | | 51 | | | | 67 |
| 2-ethyl-4-imidazole | 1 | | | 1 | 1 | | | 1 |
| Tetrabutylammonium bromide | | | 1 | | | | 2 | |
| Curability of the composition (residual coating film %) | 96 | 93 | 97 | 97 | 99 | 99 | 98 | 98 |
| Thermal degradability of the cured coating film (residual coating film %) | 6 | 6 | 5 | 7 | 98 | 97 | 98 | 98 |

## Claims

1. A polyepoxy compound containing at least two olefin oxide groups and at least one tertiary ester group per molecule and having a weight average molecular weight of 100 to 50,000, the tertiary ester group being represented by general formula (I).

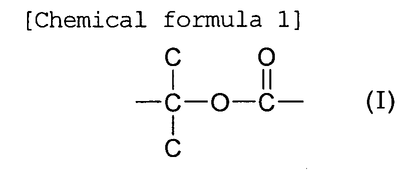

2. A polyepoxy compound according to claim 1 which is represented by general formula (II) 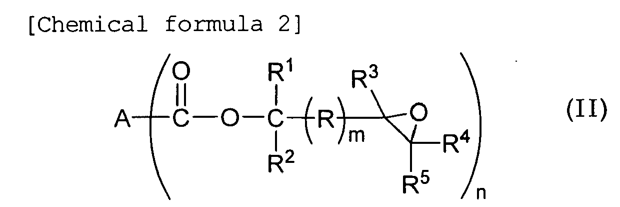 wherein n is an integer from 2 to 8, m is an integer of 0 or 1, R¹ and R² each independently represent a C₁₋₂₄ monovalent organic group, R³, R⁹ and R⁵ each independently represent a hydrogen atom, a C₁₋₆ lower alkyl group, a C₁₋₄ lower alkoxy group, a halogen atom, a cyano group, or a nitro group, R is a C₁₋₂₄ divalent organic group, and A is a C₁₋₂₄ divalent to octavalent organic group.

3. A polyepoxy compound according to claim 2 wherein R is an organic group containing ether bond(s).

4. A polyepoxy compound according to claim 1 which is at least one compound selected from the group consisting of polyepoxy compounds of the following formulas 1 to 5:

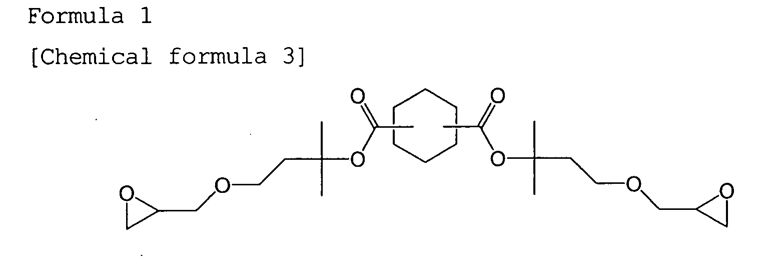

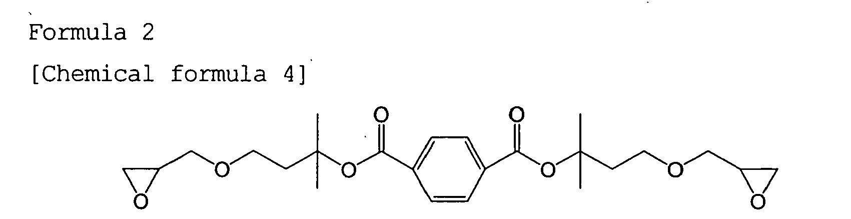

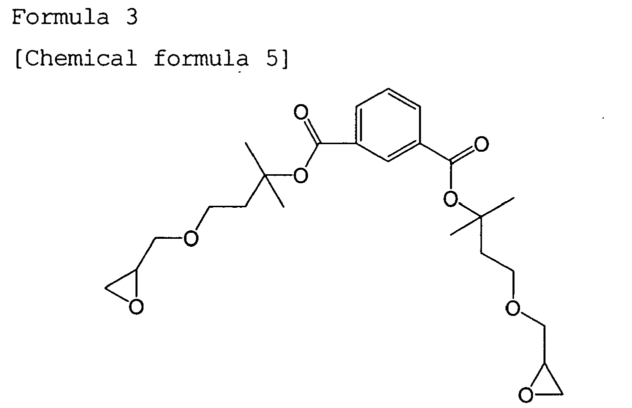

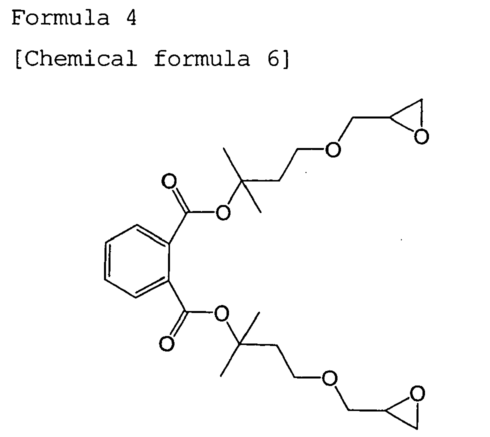

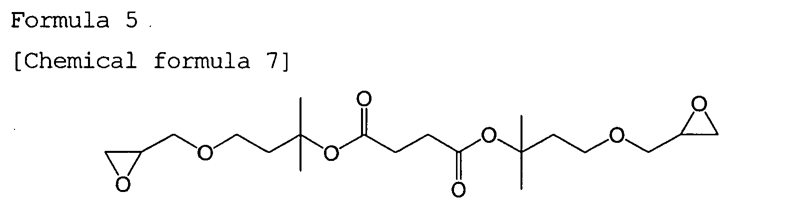

5. A method for producing a polyepoxy compound, the method comprising esterifying at least one compound selected from the group consisting of:
(a) compounds containing per molecule at least two haloformyl groups represented by general formula (III) 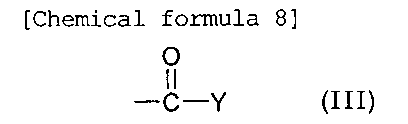
wherein Y is a halogen atom; (b) polycarboxylic acids; and (c) acid anhydrides;
with a tertiary alcohol containing an olefin bond
to produce an intermediate having olefin bonds and tertiary ester bonds,
then oxidizing the olefin bond of the intermediate to produce a compound having olefin oxide groups and tertiary ester groups.

6. A method according to claim 5 wherein the tertiary alcohol containing an olefin bond is a compound represented by general formula (IV) 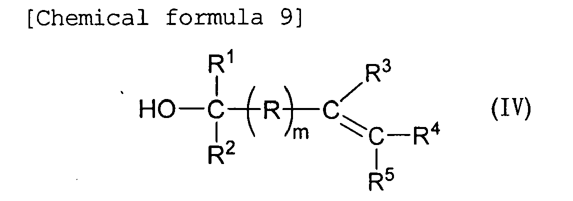 wherein n is an integer from 2 to 8, m is an integer of 0 or 1, R¹ and R² each independently represent a C₁₋₂₄ monovalent organic group, R³, R⁴ and R⁵ each independently represent a hydrogen atom, a C₁₋₆ lower alkyl group, a C₁₋₄ lower alkoxy group, a halogen atom, a cyano group, or a nitro group, and R is a C₁₋₂₄ divalent organic group.

7. A method according to claim 6 wherein the tertiary alcohol containing an olefin bond is an allyl ether compound represented by general formula (V) 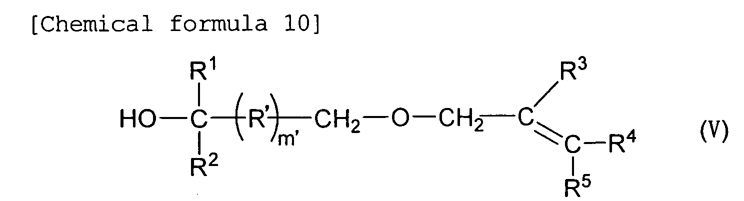 wherein m' is an integer of 0 or 1, R' is a C₁₋₂₂ divalent organic group, R¹, R², R³, R⁴ and R⁵ are as defined above.

8. A thermosetting resin composition comprising (A) a carboxyl- and/or hydroxyphenyl-containing resin, and (B) the polyepoxy compound of claim 1.

9. The thermosetting resin composition according to claim 8 wherein the resin (A) is at least one resin selected from the group consisting of:
carboxyl-containing resin (a) having a weight average molecular weight of 500 to 100,000, a glass transition point of 0 to 200°C, and an acid value of 5 to 700 mgKOH/g;
hydroxyphenyl-containing resin (b) having a weight average molecular weight of 500 to 100,000, a glass transition point of 0 to 200°C, and a hydroxyl value of 5 to 600 mgKOH/g; and
carboxyl- and hydroxyphenyl-containing resin (c) having a weight average molecular weight of 500 to 100,000, a glass transition point of 0 to 200°C, an acid value of 5 to 700 mgKOH/g, and a hydroxyl value of 5 to 600 mgKOH/g.

10. The thermosetting resin composition according to claim 8 which comprises 1 to 3,000 parts by weight of the compound (B) per 100 parts by weight of the resin (A).

11. A cured product obtained by heating and curing the thermosetting resin composition of claim 8.

12. A method for removing a cured product, the method comprising heating the whole or part of the cured product of claim 11 at a temperature higher than the heat-curing temperature to thermally cleave a tertiary ester bond derived from the polyepoxy compound (B), and removing the heated portion of the cured product by using or not using a treatment agent.

13. The method for removing the cured product of claim 12 wherein the treatment agent is an organic solvent.

14. The method for removing the cured product of claim 12 wherein the cured product is a cured coating film or cured adhesive film formed on scrap.

15. A thermosetting resin composition comprising (B) the polyepoxy compound of claim 1, and (C) a curing agent.

16. The thermosetting resin composition according to claim 15 wherein the curing agent (C) is at least one compound selected from the group consisting of acid anhydrides, polycarboxylic acids, polyphenolic compounds, nitrogen-containing compounds, cationic polymerization catalysts, and sulfur-containing compounds.

17. The thermosetting resin composition according to claim 16 wherein the acid anhydride is at least one compound selected from the group consisting of hexahydrophthalic anhydride, methylhexahydrophthalic anhydride, methylnadic anhydride, and maleic anhydride.

18. The thermosetting resin composition according to claim 16 wherein the polycarboxylic acid is at least one compound selected from the group consisting of phthalic acid, isophthalic acid, terephthalic acid, and 4,4'-biphenyldicarboxylic acid.

19. The thermosetting resin composition according to claim 16 wherein the polyphenolic compound is at least one compound selected from the group consisting of resorcinol and bisphenols.

20. The thermosetting resin composition according to claim 16 wherein the nitrogen-containing compound is at least one compound selected from the group consisting of amine compounds, amide compounds, and imidazole compounds.

21. The thermosetting resin composition according to claim 16 wherein the cationic polymerization catalyst is at least one compound selected from the group consisting of Lewis acid salts of aromatic diazonium, Lewis acid salts of aromatic sulfonium, Lewis acid salts of aromatic iodonium, and Lewis acid salts of metallocene compounds.

22. The thermosetting resin composition according to claim 15 comprising 0.1 to 500 parts by weight of curing agent (C) per 100 parts by weight of polyepoxy compound (B).

23. The cured product obtained by heating and curing the thermosetting resin composition of claim 15.

24. A method for removing a cured product comprising heating the whole or part of the cured product of claim 23 at a temperature higher than the heat-curing temperature to thermally cleave a tertiary ester bond derived from the polyepoxy compound (B) and removing the heated portion of the cured product by using or not using a treatment agent.

25. The method according to claim 24 wherein the treatment agent is an organic solvent.

26. The method according to claim 24 wherein the cured product is a cured coating film or cured adhesive film formed on scrap.
